(19) Europäisches Patentamt
European Patent Office
Office européen des brevets

(11) Veröffentlichungsnummer: **0 344 109 B1**

# (12) EUROPÄISCHE PATENTSCHRIFT

(45) Veröffentlichungstag der Patentschrift: **27.04.94**

(51) Int. Cl.[5]: **C07C 50/36**, G03C 1/73, C07C 323/22, C07D 307/12, C07C 225/32, C07D 295/10, C07C 317/24, C07C 69/95, C07C 255/56

(21) Anmeldenummer: **89810363.5**

(22) Anmeldetag: **18.05.89**

Die Akte enthält technische Angaben, die nach dem Eingang der Anmeldung eingereicht wurden und die nicht in dieser Patentschrift enthalten sind.

## (54) Substituierte Naphthacen-5,12-dione und deren Verwendung.

(30) Priorität: **27.05.88 CH 2008/88**
**19.07.88 CH 2757/88**

(43) Veröffentlichungstag der Anmeldung:
**29.11.89 Patentblatt 89/48**

(45) Bekanntmachung des Hinweises auf die Patenterteilung:
**27.04.94 Patentblatt 94/17**

(84) Benannte Vertragsstaaten:
**CH DE FR GB IT LI NL**

(56) Entgegenhaltungen:
**EP-A- 112 798**
**EP-A- 0 109 360**
**EP-A- 0 195 743**
**US-A- 4 039 413**
**US-A- 4 046 577**

**CHEMICAL ABSTRACTS SUBSTANCE, ELEVENTH COLLECTIVE INDEX, Band 96-105, 1982-1986, Seite 41661CS, Columbus, Ohio, US; & JP-A-57 187 870**

(73) Patentinhaber: **CIBA-GEIGY AG**
**Klybeckstrasse 141**
**CH-4002 Basel(CH)**

(72) Erfinder: **Fischer, Walter, Dr.**
**Vogesenstrasse 77**
**CH-4153 Reinach(CH)**
Erfinder: **Baumann, Marcus, Dr.**
**Arlesheimerstrasse 18**
**CH-4053 Basel(CH)**
Erfinder: **Finter, Jürgen, Dr.**
**Zasiusstrasse 100**
**D-7800 Freiburg(DE)**
Erfinder: **Kvita, Vratislav, Dr.**
**Vogesentrasse 71**
**CH-4153 Reinach(CH)**
Erfinder: **Mayer, Carl W., Dr.**
**Steingrubenweg 224**
**CH-4125 Riehen(CH)**
Erfinder: **Wernet, Wolfgang, Dr.**
**Schillerstrasse 40**
**D-7800 Freiburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CHEMICAL ABSTRACTS, Band 96, Nr. 21, 24. Mai 1982, Seite 678, Zusammenfassung Nr. 180914q, Columbus, Ohio, USA; L. K. Bee et al: "Diels-Alder reactions of 7,7-dichloro-3,4-dimethylenebicyclo[4.1.0] heptane with quinones. Synthesis of tetracenequinones"

CANADIAN JOURNAL OF CHEMISTRY, Band 58, Nr. 12, 15. Juni 1980, Seiten 1161-1167, National Research Council of Canada, Ottawa, CA; J. LADURANTY et al: "Capture du paradiméthoxyorthoquinodiméthane: synthèses du quinones et d'unintermédiaire de la daunomycinone"

CHEMICAL ABSTRACTS, Band 91, Nr. 9, 27. August 1979, Seite 575, Zusammenfassung Nr. 74384d, Columbus, Ohio, US; T. KAMETANI: "Tetracyclic adducts from 6-methoxybenzocyclobutenol and naphthoquinones"; & JP-A-79 39 065

CHEMICAL ABSTRACTS, Band 86, Nr. 25, 20. Juni 1977, Seite 597, Zusammenfassung Nr. 189773u, Columbus, Ohio, US; J. FREIMANIS et al: "Synthesis and some properties of 2-isopropyltetracene"

TETRAHEDRON LETTERS, Band 25, Nr. 42, 1984, Seiten 4833-4836, Pergamon Press Ltd., Oxford, GB; R. N. WARRENER et al: "Linear annelation of tricyclic quinones by site selective reaction with dienes"

CHEMICAL ABSTRACTS, Band 84, Nr. 23, 7. Juni 1976, Seite 133, Zusammenfassung Nr. 160796b, Columbus, Ohio, US; J. C. DOUBLE et al: "Evalution of the binding of some substituted anthraquinones and naphthacenequinones"

SYNTHESIS, Nr. 7, Juli 1973, Seiten 416-417, Georg Thieme Verlag, Stuttgart, DE; J. F. W. McOMIE et al: "Aromatic annelation using omicron-quinodimethanes as reactive intermediates"

JOURNAL OF THE AMERICAN CHEMICAL SOCIETY, Band 95, Nr. 14, 11. Juli 1973, Seiten 4606-4610, Washington DC, US; H. E. ZIMMERMAN et al: "Photochemical rearrangement effected by triplet excitation transmitted through a high energy moiety. Mechanistic and exploratory organic photochemistry. LXXXX"

BULLETIN DE LA SOCIETE CHIMIOUE DE FRANCE, Nr. 3, März 1973, Seiten 1154-1159, Paris, FR; A. VERINE et al: "No 190. - Synthèses de quinones polycyliques par carbanions"

CHEMICAL ABSTRACTS, Band 76, Nr. 13, 27. März 1972, Seite 72, Zusammenfassung Nr. 73712n, Columbus, Ohio, US; K. KONISHI et al: "Influence of beta-substituents on the sublimation properties of anthraquinonoid disperse dyes"

JUSTUS LIEBIGS: "Annalen der Chemie", Band 754, 1971, Seite 64-89, Verlag Chemie GmbH, Weinheim, DE; E. MÜLLER et al: "Synthesen von Chinonen"

CHEMICAL ABSTRACTS, Band 74, Nr. 19, 10. Mai 1971, Zusammenfassung Nr. 93441k, Columbus, Ohio, US; V. N. KOSTYLEV et al: "Spectral manifestation of the photochromism of 1-phenoxyanthraquinone and 10-phenoxynaphthacenequinone"

TETRAHEDRON, Band 26, 1970, Seiten 5465-5478, Pergamon Press, Oxford, GB; E. CLAR et al: "The electronic interaction in benzologues of pyrene"

ANALES DE OUIMICA, Band 81, Nr. 2, 1985, Seiten 133-138, Madrid, ES; F. FARINA et al: "Hidroxiquinonas policiclicas. XX. Sintesis de hidroxiquinonas tetraciclicas por reaccion de Diels-Alder entre omicron-quinodimetanos y derivados naftoquinonicos funcionalizados"

## Beschreibung

Die vorliegende Erfindung betrifft substituierte Naphthacen-5,12-dione und deren Verwendung als Photoinitiatoren oder Sensibilisatoren zur Photopolymerisation von Verbindungen mit mindestens einer polymerisierbaren oder dimerisierbaren ethylenisch ungesättigten Doppelbindung, oder zur stromlosen Metallabscheidung auf der Oberfläche von Polymeren, die Hydroxyalkylgruppen enthalten.

Es sind bis heute nur wenige substituierte Naphthacen-5,12-dione bekannt geworden. Sie stellen wertvolle Zwischenprodukte zur Herstellung von tetrachalkogenierten Tetracenen dar. Solche tetrachalkogenierten Tetracene bilden mit Elektronendonoren elektrisch leitende Charge-Transferkomplexe.In der US-PS 4,617,151 sind z.B. 2- bzw. 2,3-Carbonsäuren und Carbonsäurederivate von Naphthacen-5,12-dion beschrieben. In der DE-OS 3,635,124 sind 2,7- und 2,8-Difluornaphthacen-5,12-dion erwähnt.

In der Japanischen Patentveröffentlichung 49-81440 sind 1-Hydroxyl- bzw. 1-Amino-naphthacen-5,12-dione, die in 4-Stellung und gegebenenfalls in 3-Stellung substituiert sind, als fluoreszierende Dispersionsfarbstoffe beschrieben. F.F. y T. Torres beschreibt in Annales die Quimica, Ser. C, 81(2), S. 133-138 (1982) die Herstellung von 2-Hydroxy-, 2-Methoxy-, 2-Ethoxy-, 2-(2'Hydroxyethoxy)naphthacen-5,12-dion. Naphthacen-5,12-dione mit Methoxygruppen in den 6-, 7-, 8- und 9-Stellungen sind z.B. beschrieben in D.C.C. Smith, J. Chem. Soc., S. 673 (1962), J.F.W. McOmie et al., Synthesis, S. 416 (1973), und J. Laduranty et al., Can. J. Chem., Vol. 58, S. 1161 (1980). 7-Methyl-naphthacen-5,12-dion ist von L.K. Bee et al. in J. Chem. Res. (M), S. 4301 (1981) beschrieben. 2-Isopropyl-naphthacen-5,12-dion ist in CA 86, 189773u (1977) erwähnt.

Gegenstand vorliegender Erfindung sind Verbindungen der Formel I

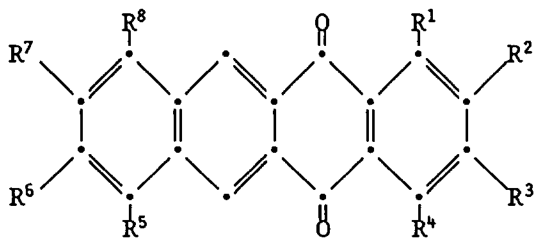

(I),

worin $R^5$ und $R^8$ H sind und

a) $R^1$, $R^2$, $R^3$, $R^4$ H sind,

und $R^6$ und $R^7$ je für H stehen und mindestens einer der Reste $R^6$ und $R^7$ unabhängig voneinander einen Rest aus der Gruppe unsubstituiertes oder mit Halogen, -CN, Furfuryl, $-NR^9R^{10}$, $-OR^9$, $-SR^9$ oder $-COOR^9$ substituiertes $C_1$-$C_{20}$-Alkyl$(X)_p$ mit Ausnahme von Methoxy, $C_2$-$C_{18}$-Alkenyl$(X)_p$, $C_2$-$C_{18}$-Alkinyl$(X)_p$, $C_3$-$C_8$-Cycloalkyl$(X)_p$, ($C_1$-$C_{12}$-Alkyl)-$C_3$-$C_8$-cycloalkyl$(X)_p$, $C_3$-$C_8$-Cycloalkyl-$C_rH_{2r}(X)_p$, ($C_1$-$C_{12}$-Alkyl)-$C_3$-$C_8$-cyclo alkyl-$C_rH_{2r}(X)_p$, Phenyl$(X)_p$, ($C_1$-$C_{12}$-Alkyl)phenyl$(X)_p$, Phenyl-$C_rH_{2r}(X)_p$,($C_1$-$C_{12}$-Alkyl)phenyl-$C_rH_{2r}(X)_p$ darstellen,

r für 1 oder 2 und p für 1 stehen und X -O-, -SO- oder $-SO_2$-bedeutet, oder $R^6$, $R^7$ unabhängig einen Rest aus der Gruppe Halogen, $-NO_2$, $-CF_3$, -CN, $-NR^9R^{10}$, $-COOR^9$, $-CONR^9R^{10}$, -COCl, -SH, -Si($C_1$-$C_4$-Alkyl)$_3$ oder $-O(C_mH_{2m}-O)_nR^{11}$ bedeuten,

oder $R^6$ und $R^7$ zusammen für -CO-O-CO-oder $-CO-NR^9-CO-$ stehen,

$R^9$ und $R^{10}$ unabhängig voneinander H, $C_1$-$C_{12}$-Alkyl, Phenyl oder $(C_mH_{2m}-O)_qR^{11}$ oder $R^9$ und $R^{10}$ zusammen Tetramethylen, Pentamethylen, 3-Oxapentylen oder $-CH_2CH_2NR^9CH_2CH_2-$ bedeuten, $R^{11}$ H oder $C_1$-$C_{12}$-Alkyl ist,

m für eine Zahl von 2 bis 4, n für eine Zahl von 2 bis 20 und q für eine Zahl von 1 bis 20 stehen, oder

b) $R^1$ bis $R^4$, $R^6$ und $R^7$ unabhängig voneinander H oder einen der zuvor definierten Reste einschliesslich Methoxy darstellen, wobei $R^1$ und $R^4$ auch Reste mit X in der Bedeutung von -S- sind und mindestens eines von $R^1$ bis $R^4$ sowie $R^6$ und $R^7$ einen Rest bedeuten, mit Ausnahme von $R^2$ und $R^6$ oder $R^7$ gleich -F, oder

c) $R^6$ und $R^7$ für H stehen, mindestens eines von $R^1$, $R^2$, $R^3$ und $R^4$ einen Rest bedeuten,

und $R^1$ und $R^4$ unabhängig voneinander H oder einen Rest aus der Gruppe $-NO_2$, $-CF_3$, -CN, $-COOR^9$, $-CONR^9R^{10}$, -COCl, -Si($C_1$-$C_4$-Alkyl)$_3$ $-S(C_mH_{2m}-O)_nR^{11}$, $-O(C_mH_{2m}-O)_nR^{11}$, unsubstituiertes oder wie zuvor unter a) definiert substituiertes $C_1$-$C_{20}$-Alkyl-$X^1$-; $C_2$-$C_{18}$-Alkenyl$(X^1)_p$, $C_2$-$C_{18}$-Alkinyl$(X^1)_p$, $C_3$-$C_8$-Cycloalkyl$(X^1)_p$, ($C_1$-$C_{12}$-Alkyl)-$C_3$-$C_8$-cycloalkyl$(X^1)_p$, $C_3$-$C_8$-Cycloalkyl-$C_rH_{2r}(X^1)_p$,($C_1$-$C_{12}$-Alkyl)-$C_3$-$C_8$-cycloalkyl-$C_rH_{2r}(X^1)_p$; Phenyl$(X^1)_p$

oder ($C_1$-$C_{12}$-Alkyl)phenyl$\{X^1\}_p$, Phenyl-$C_rH_{2r}\{X\}_p$, ($C_1$-$C_{12}$-Alkyl)phenyl-$C_rH_{2r}\{X\}_p$; wie zuvor unter a) definiert substituiertes $C_1$-$C_{20}$-Alkylthio, Phenyloxy, ($C_1$-$C_{12}$-Alkyl)phenyloxy oder mit Halogen, -CN, -$NR^9R^{10}$, -$SR^9$, -$OR^9$ oder $COOR^9$ substituiertes $C_1$-$C_{20}$-Alkoxy darstellen; wobei $R^1$ nicht -COOH ist, wenn $R^2$, $R^3$ und $R^4$ H bedeuten, und $R^2$ und $R^3$ unabhängig voneinander H oder einen Rest aus der Gruppe unsubstituiertes oder wie zuvor unter a) definiert substituiertes $C_5$-$C_{20}$-Alkyl, $C_3$-$C_{20}$-Alkoxy, $C_1$-$C_{20}$-Alkyl-$X^1$-; Phenyl-X-, ($C_1$-$C_{12}$-Alkyl)phenyl$\{X\}_p$, ($C_1$-$C_{12}$-Alkyl)phenyl-$C_rH_{2r}\{X\}_p$, Phenyl-$C_rH_{2r}\{X\}_p$, $C_2$-$C_{20}$-Alkenyl$\{X\}_p$, $C_2$-$C_{20}$-Alkinyl$\{X\}_p$, $C_3$-$C_8$-Cycloalkyl$\{X\}_p$,($C_1$-$C_{12}$-Alkyl)-$C_3$-$C_8$-cycloalkyl$\{X\}_p$, $C_3$-$C_8$-Cycloalkyl-$C_rH_{2r}\{X\}_p$ oder ($C_1$-$C_{12}$-Alkyl)-$C_3$-$C_8$-cycloalkyl-$C_rH_{2r}\{X\}_p$,
oder eines von $R^2$ und $R^3$ oder $R^2$ und $R^3$ einen Rest aus der Gruppe mit Halogen, -CN, -$NR^9R^{10}$, $OR^9$, -$SR^9$, -$COOR^9$ substituiertes $C_1$-$C_4$-Alkyl oder $C_1$-$C_2$-Alkoxy mit Ausnahme von 2-Hydroxyethyl oder eines von $R^2$ und $R^3$ oder $R^2$ und $R^3$ einen Rest aus der Gruppe SH, -$NO_2$, -$CF_3$, -CN, -$NR^9R^{10}$, -Si($C_1$-$C_4$-Alkyl)$_3$ oder -O$\{C_mH_{2m}$-O$\}_nR^{11}$ darstellen;
oder eines von $R^2$ und $R^3$ Halogen, -$COOR^9$, -$CONR^9R^{10}$ oder -COCl und das andere von $R^2$ und $R^3$ einen wie unter a) definierten Rest darstellen, oder das andere von $R^2$ und $R^3$ H ist, wenn mindestens eines von $R^1$ und $R^4$ einen der zuvor definierten Reste darstellt;
oder $R^2$ und $R^3$ zusammen -CO-O-CO- oder -CO-$NR^9$-CO- und mindestens eines von $R^1$ und $R^4$ einen der zuvor definierten Reste darstellen, oder $R^2$ und $R^3$ zusammen unabhängig voneinander $C_1$-$C_2$-Alkoxy sind, wobei $R^9$, $R^{10}$, $R^{11}$, X, m, n, p, q, r und s, die unter a) angegebenen Bedeutungen haben und $X^1$ -SO- oder -$SO_2$- darstellt, oder
d) $R^6$ und $R^7$ H bedeuten und $R^1$ unsubstituiertes $C_1$-$C_{20}$-Alkylthio, $C_1$-$C_{20}$-Alkoxy, Phenyloxy, Phenylthio, $C_1$-$C_{12}$-Alkylphenyloxy, $C_1$-$C_{12}$-Alkylphenylthio, oder wie unter a) definiert substituiertes Phenylthio oder $C_1$-$C_{12}$-Alkylphenylthio darstellen.

Bedeuten im Rahmen der vorangehenden Definitionen $R^1$ bis $R^4$, $R^6$ und $R^7$ Alkyl$\{X\}_p$, so kann die Alkylgruppe linear oder verzweigt sein und vorzugsweise 1 bis 18, besonders 1 bis 12 und insbesondere 1 bis 6 C-Atome enthalten. Beispiele für Alkylgruppen sind Methyl, Ethyl und die Isomeren von Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl und Eicosyl.

Bedeuten im Rahmen der vorangehenden Definitionen $R^1$ bis $R^4$, $R^6$ und $R^7$ Alkenyl$\{X\}_p$ so kann die Alkenylgruppe linear oder verzweigt sein und vorzugsweise 3 bis 12, besonders 3 bis 6 C-Atome enthalten. Das Alkenyl enthält bevorzugt terminale Doppelbindungen. Einige Beispiele sind Ethenyl, Allyl, Prop-1-en-1- oder -2-yl, But-1-en-1- oder -2- oder -3- oder -4-yl, But-2-en-1- oder -2-yl, Pent-1-en- oder Pent-2-en-1-oder -2- oder -3- oder -4- oder -5-yl, Hex-1-en- oder Hex-2-en- oder Hex-3-en-1- oder -2- oder -3- oder -4- oder -5- oder -6-yl, Heptenyl, Octenyl, Nonenyl, Decenyl, Undecenyl, Dodecenyl, Tetracenyl, Hexadecenyl und Octadecenyl.

Bedeuten im Rahmen der vorangehenden Definition $R^1$ bis $R^4$, $R^6$ und $R^7$ $C_2$-$C_{18}$-Alkinyl$\{X\}_p$, so kann die Alkinylgruppe linear oder verzweigt sein und vorzugsweise 3 bis 12, besonders 3 bis 6 C-Atome enthalten. Die Dreifachbindung befindet sich vorzugsweise in terminaler Stellung. Einige Beispiele sind Ethinyl, Propargyl, But-1-in-3- oder -4-yl, But-2-in-1-yl, Pent-1-in-3- oder -4- oder -5-yl, Hex-1-in-3- oder -4- oder -5- oder -6-yl, Hex-2-in-1- oder -4- oder -5- oder -6-yl, Hex-3-in-1- oder -2-yl, Heptinyl, Octinyl, Noninyl, Decinyl, Undecinyl und Dodecinyl.

Bedeuten $R^1$ bis $R^4$, $R^6$ und $R^7$ im Rahmen der vorangehenden Definitionen $C_3$-$C_8$-Cycloalkyl$\{X\}_p$, so kann es sich um Cyclopropyl, Cyclobutyl, Cyclopentyl, Cyclohexyl, Cycloheptyl oder Cyclooctyl handeln. X steht bevorzugt für -O-. Bevorzugt ist $C_3$-$C_6$-Cycloalkyl.

Bedeuten $R^1$ bis $R^4$, $R^6$ und $R^7$ im Rahmen der vorangehenden Definitionen $C_3$-$C_8$-Cycloalkyl-$C_rH_{2r}\{X\}_p$, ($C_1$-$C_{12}$-Alkyl)-$C_3$-$C_8$-cycloalkyl$\{X\}_p$ oder ($C_1$-$C_{12}$-Alkyl)-$C_3$-$C_8$-cycloalkyl-$C_rH_{2r}\{X\}_p$, so kann die Alkylgruppe linear oder verzweigt sein und vorzugsweise 1 bis 6, besonders 1 bis 4 C-Atome enthalten. In der -$C_rH_{2r}$-Gruppe steht r bevorzugt für 1. X steht bevorzugt für -O-. Die Cycloalkylgruppe stellt in diesen Resten bevorzugt $C_3$-$C_6$-Cycloalkyl dar.

Bedeuten $R^1$ bis $R^4$, $R^6$ und $R^7$ im Rahmen der vorangehenden Definitionen ($C_1$-$C_{12}$-Alkyl)phenyl$\{X\}_p$, Phenyl-$C_rH_{2r}\{X\}_p$ oder ($C_1$-$C_{12}$-Alkyl)phenyl$\{X\}_p$,so kann die Alkylgruppe linear oder verzweigt sein und vorzugsweise 1 bis 6, besonders 1 bis 4 C-Atome enthalten. In der -$C_rH_{2r}$-Gruppe steht r bevorzugt für 1.

$R^1$ bis $R^4$, $R^6$ und $R^7$ können im Rahmen der vorangehenden Definitionen unsubstituiert oder ein- oder mehrfach, bevorzugt ein- bis dreifach, insbesondere ein-oder zweifach substituiert sein. Ist der Substituent Halogen, so handelt es sich bevorzugt um -F, -Cl oder -Br. Ist der Substituent -$NR^9R^{10}$, so bedeuten $R^9$ und $R^{10}$ bevorzugt unabhängig voneinander H oder $C_1$-$C_4$-Alkyl und besonders Methyl oder Ethyl. Ist der Substituent -$OR^9$,
-$SR^9$ oder -$COOR^9$, so stellt $R^9$ bevorzugt H, $C_1$-$C_4$-Alkyl oder
-O$\{C_mH_{2m}$-O$\}_nR^{11}$ dar, worin $R^{11}$ H oder $C_1$-$C_4$-Alkyl bedeutet und m für 2 oder 3 und n für 1 bis 12

stehen.

Sind $R^1$ bis $R^4$, $R^6$ und $R^7$ im Rahmen der vorangehenden Definitionen Halogen, so handelt es sich besonders um -F, -Cl oder -Br.

Sind $R^1$ bis $R^4$, $R^6$ und $R^7$ im Rahmen der vorangehenden Definitionen $-NR^9R^{10}$ oder $-CONR^9R^{10}$, so sind $R^9$ und $R^{10}$ unabhängig voneinander bevorzugt $C_1$-$C_6$-Alkyl oder $R^9$ und $R^{10}$ zusammen bevorzugt Tetra- oder Pentamethylen oder 3-Oxapentylen.

Sind $R^1$ bis $R^4$, $R^6$ und $R^7$ im Rahmen der vorangehenden Definitionen -Si($C_1$-$C_4$-Alkyl)$_3$, so handelt es sich bevorzugt um -Si($C_1$ oder $C_2$-Alkyl)$_3$ und insbesondere um Trimethylsilyl.

Sind $R^1$ bis $R^4$, $R^6$ und $R^7$ im Rahmen der vorangehenden Definitionen $-O\{C_mH_{2m}-O\}_nR^{11}$, so stellt $R^{11}$ bevorzugt H oder $C_1$-$C_4$-Alkyl dar, m steht bevorzugt für 2 oder 3 und n bevorzugt für 1 bis 12, besonders 1 bis 6.

Benachbarte Reste für die Gruppen -CO-O-CO- und $-CO-NR^9-CO-$ sind besonders $R^2$ und $R^3$ und/oder $R^6$ und $R^7$.

$R^9$ und $R^{10}$ enthalten als Alkyl bevorzugt 1 bis 6, besonders 1 bis 4 C-Atome und sind insbesondere Methyl oder Ethyl. Das Alkyl kann linear oder verzweigt sein.

Bedeuten $R^9$ und $R^{10}$ die Gruppe $\{C_mH_{2m}-O\}_nR^{11}$, so stellen bevorzugt m 2 oder 3, n 1 bis 12, besonders 2 bis 6 und $R^{11}$ H oder $C_1$-$C_4$-Alkyl dar.

In einer bevorzugten Ausführungsform stehen $R^9$ und $R^{10}$ unabhängig voneinander für H, $C_1$-$C_4$-Alkyl, Phenyl, oder $R^9$ und $R^{10}$ zusammen für Tetramethylen, Pentamethylen oder 3-Oxapentylen.

Bevorzugte Ausführungsformen von Verbindungen der Formel I sind solche, worin a) $R^4$ oder $R^1$ und $R^4$ für H stehen, oder b) $R^2$ oder $R^3$ oder $R^2$ und $R^3$ einen Substituenten darstellen, oder c) $R^1$,$R^2$ und $R^3$ oder $R^1$ und $R^2$ oder $R^1$ und $R^3$ einen Substituenten darstellen, oder d) $R^2$ und $R^3$ oder $R^2$ oder $R^3$ einen Substituenten und $R^6$ oder $R^7$ oder $R^6$ und $R^7$ einen Substituenten darstellen, oder e) $R^1$,$R^2$ und $R^3$ oder $R^1$ und $R^2$ oder $R^1$ und $R^3$ einen Substituenten und $R^4$ H bedeuten und $R^6$ oder $R^7$ oder $R^6$ und $R^7$ H oder einen Substituenten darstellen. Bevorzugte Verbindungen der Formel I sind auch solche, worin $R^4$ für H steht.

Eine bevorzugte Untergruppe von Verbindungen der Formel I sind solche, worin $R^1$ bis $R^4$ H bedeuten; mindestens einer der Reste $R^6$ oder $R^7$ einen Substituenten aus der Gruppe unsubstituiertes oder mit F, Cl, Br, -CN, $-NR^9R^{10}$, $-OR^9$, $-SR^9$ oder $-COOR^9$ substituiertes $C_1$-$C_{18}$ Alkyl$\{X\}_p$, Phenyl$\{X\}_p$, ($C_1$-$C_6$-Alkyl)-phenyl$\{X\}_p$, Benzyl$\{X\}_p$, ($C_1$-$C_6$-Alkyl)benzyl$\{X\}_p$;
unsubstituiertes $C_3$-$C_{12}$-Alkenoxy oder $C_3$-$C_6$-Alkinoxy, deren Alken- bzw. Alkingruppe nicht an das O-Atom gebunden ist; oder -F, -Cl, -Br, $-NO_2$, $-CF_3$, -CN, $-NR^9R^{10}$, $COOR^9$, $-CONR^9R^{10}$, $-Si(CH_3)_3$ oder $-O\{C_2H_4O\}_nR^{11}$ darstellt; $R^{11}$ H oder $C_1$-$C_4$-Alkyl ist;
X -O-, -SO- oder $-SO_2$-, p 1 und n 2 bis 20 darstellen, und wobei $R^9$ und $R^{10}$ je für H, $C_1$-$C_6$-Alkyl oder $R^9$ und $R^{10}$ zusammen Tetra-oder Pentamethylen, 3-Oxapentylen oder $-CH_2CH_2N(C_1$-$C_6$-Alkyl)$CH_2CH_2$-stehen.

Eine andere bevorzugte Untergruppe von Verbindungen der Formel I sind solche, worin $R^4$ H ist;
mindestens einer der Reste $R^1$, $R^2$ und $R^3$ sowie mindestens einer der Reste $R^6$ und $R^7$ einen Substituenten aus der Gruppe unsubstituiertes oder mit F, Cl, Br, -CN, $-NR^9R^{10}$, $-OR^9$, $-SR^9$ oder $-COOR^9$ substituiertes $C_1$-$C_{18}$ Alkyl$\{X\}_p$ einschliesslich Methyl und Methoxy, Phenyl$\{X\}_p$,($C_1$-$C_6$-Alkyl)phenyl$\{X\}_p$, Benzyl$\{X\}_p$, ($C_1$-$C_6$-Alkyl)benzyl$\{X\}_p$;
unsubstituiertes $C_3$-$C_{12}$-Alkenoxy oder $C_3$-$C_6$-Alkinoxy, deren Alken- bzw. Alkingruppe nicht an das O-Atom gebunden ist; oder -F, -Cl, -Br, $-NO_2$, $-CF_3$, -CN, $-NR^9R^{10}$, $-COOR^9$, $-CONR^9R^{10}$, $-Si(CH_3)_3$, $-O\{C_2H_4O\}_nR^{11}$ darstellt; $R^{11}$ H oder $C_1$-$C_4$-Alkyl ist;
X -O-, -SO- oder $-SO_2$- p 1 und n 2 bis 20 darstellen; und
$R^9$ und $R^{10}$ je für H, $C_1$-$C_6$-Alkyl oder $R^9$ und $R^{10}$ zusammen Tetra- oder Pentamethylen, 3-Oxapentylen oder $-CH_2CH_2N(C_1$-$C_6$-Alkyl)$CH_2CH_2$- stehen.

Eine bevorzugte Untergruppe von Verbindungen der Formel I sind auch solche, worin $R^6$ und $R^7$ H sind;
$R^1$ und $R^4$ unabhängig voneinander H oder einen Rest aus der Gruppe $-NO_2$, $-CF_3$, -CN, $-COOR^9$, $-CONR^9R^{10}$, $-Si(CH_3)_3$, $-S\{C_2H_4-O\}_nR^{11}$, $-O\{C_2H_4-O\}_nR^{11}$ bedeuten, wobei $R^9$ und $R^{10}$ je für H, $C_1$-$C_6$-Alkyl, und $R^9$ und $R^{10}$ zusammen Tetramethylen, Pentamethylen oder 3-Oxapentylen stehen;
$R^2$ und $R^3$ unabhängig voneinander H oder einen Rest aus der Gruppe $C_5$-$C_{18}$-Alkyl, $C_3$-$C_{18}$-Alkoxy, $C_1$-$C_{18}$-Alkyl-$X^1$-, Phenyl-X-, ($C_1$-$C_6$-Alkyl)phenyl-X-, Benzyl$\{X\}_p$ oder ($C_1$-$C_6$-Alkyl)benzyl$\{X\}_p$ mit $X^1$ gleich -SO- oder $-SO_2$ und p gleich 1, die unsubstituiert oder mit -F, -Cl, -Br, -CN, $-NR^9R^{10}$, $-OR^9$, $-SR^9$ oder $-COOR^9$ substituiert sind, oder unsubstituiertes $C_3$-$C_{12}$-Alkoxy oder $C_3$-$C_{12}$-Alkinoxy, deren Alkan- bzw. Alkingruppe nicht an das O-Atom gebunden ist; oder mit -F, -Cl, -Br, -CN, $-NR^9R^{10}$, $-SR^9$ oder $-OR^9$ substituiertes $C_1$-$C_4$-Alkyl oder $C_1$-$C_2$-Alkoxy; oder -F, -Cl, -Br, $-NO_2$, $-CF_3$, -CN, $-NR^9R^{10}$, $-Si(CH_3)_3$, oder $\{OC_2H_4O\}_nR^{11}$ mit $R^{11}$ gleich H oder $C_1$-$C_4$-Alkyl; oder eines von $R^2$ und $R^3$ -F, -Cl, $-COOR^9$ oder

$-CONR^9R^{10}$ sind und das andere von $R^2$ und $R^3$ ein wie in Anspruch 8 für $R^6$ definierter Substituent sind, oder das andere von $R^2$ und $R^3$ H ist, wenn $R^1$ ein Substituent ist; oder $R^2$ und $R^3$ $-COOR^9$, $-CONR^9R^{10}$, oder zusammen -CO-O-CO- oder $-CO-NR^9-CO-$ sind, wenn $R^1$ die zuvor als Substituent angegebenen Bedeutungen hat; oder $R^2$ und $R^3$ unabhängig Methoxy oder Ethoxy sind; $R^9$ und $R^{10}$ je für H, $C_1$-$C_6$-Alkyl oder $R^9$ und $R^{10}$ zusammen für Tetramethylen, Pentamethylen, 3-Oxapentylen oder $-CH_2CH_2N(C_1$-$C_6$-Alkyl)$CH_2CH_2$-stehen und n 2 bis 12 ist.

Eine weitere bevorzugte Untergruppe sind solche Verbindungen der Formel I, worin $R^6$ und $R^7$ H sind und $R^1$ unsubstituiertes $C_1$-$C_{12}$-Alkylthio, $C_1$-$C_{12}$-Alkoxy, Phenyloxy, ($C_1$-$C_6$-Alkyl)phenyloxy oder ($C_1$-$C_6$-Alkyl)phenylthio oder mit -F, -Cl, -OH, $C_1$-$C_4$-Alkoxy oder $C_1$-$C_4$-Alkylthio substituiertes Phenylthio oder ($C_1$-$C_6$-Alkyl)phenylthio darstellt.

$R^2$ und $R^3$ stellen als substituiertes Alkyl insbesondere mit -CN oder -COO($C_1$-$C_{12}$-Alkyl) substituiertes $C_1$-$C_6$-, besonders $C_1$- oder $C_2$-Alkyl dar.

Eine besonders bevorzugte Untergruppe sind solche Verbindungen der Formel I, worin $R^1$ H, $-NO_2$, $-CF_3$ oder -COO($C_1$-$C_4$-Alkyl) ist; $R^4$ H bedeutet; $R^3$, $R^6$ und $R^7$ H sind und $R^2$ für $C_3$ bis $C_{18}$-Alkoxy, $C_3$-$C_{12}$-Hydroxyalkyl, $C_3$-$C_6$-Dihydroxyalkyl; $C_3$-$C_{12}$-Alkenoxy oder $C_3$-$C_6$-Alkinoxy, bei dem das -O-Atom nicht an die Alken- bzw. Alkingruppe gebunden ist, $-O\{CH_2CH_2-O\}_nR^{11}$ mit n = 2 bis 12 und $R^{11}$ gleich H oder $C_1$-$C_6$-Alkyl; unsubstituiertes oder mit -F, -Cl, $C_1$-$C_4$-Alkyl, $C_1$-$C_4$-Alkoxy oder $C_1$-$C_4$-Alkylthio substituiertes Phenyloxy; $-NR^9R^{10}$ mit $R^9$ und $R^{10}$ gleich $C_1$-$C_6$-Alkyl, oder zusammen Pentamethylen oder 3-Oxapentylen; $C_1$-$C_6$-Alkyl-SO-, $C_1$-$C_6$-Alkyl-$SO_2$-, Phenyl-SO- oder Phenyl-$SO_2$-; mit -CN oder -COO($C_1$-$C_6$-Alkyl) substituiertes $C_1$-$C_4$-Alkyl; oder -CN oder $-CF_3$ darstellt;
$R^2$ und $R^3$ unabhängig $C_1$-$C_2$-Alkoxy, $\{O-CH_2CH_2)-_2OR^{11}$ mit $R^{11}$ gleich H oder $C_1$-$C_6$-Alkyl und n gleich 2 bis 12; oder -F, -Cl, -Br darstellen oder die zuvor für $R^2$ angegebenen Bedeutungen haben; sowie $R^6$ und $R^7$ unabhängig H, -F, -Cl, -Br, -CN, $-CF_3$, $-Si(CH_3)_3$, $-NO_2$, $C_1$-$C_{12}$-Alkoxy oder -COO($C_1$-$C_6$-Alkyl) bedeuten, mit Ausnahme von $R^2$ und $R^6$ oder $R^7$ gleich -F und $R^1$ und $R^4$ gleich H; oder $R^1$ bis $R^4$ H sind, $R^6$ oder $R^7$ H und $R^7$ oder $R^6$, oder $R^6$ und $R^7$ unabhängig voneinander -F, -Cl, -Br, -CN, $-NO_2$, $-CF_3$, $-Si(CH_3)_3$, $-NO_2$, -COO($C_1$-$C_4$-Alkyl) oder $C_1$-$C_{12}$-Alkoxy darstellen.

Die Verbindungen der Formel I können hergestellt werden, indem man nach Friedel-Crafts ein Naphthalindicarbonsäureanhydrid der Formel II

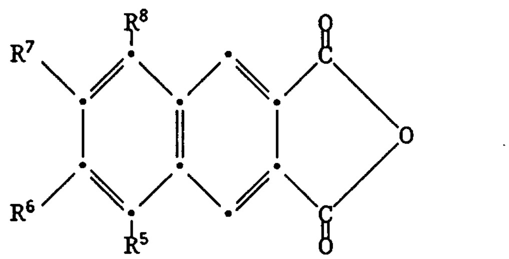

(II)

in Gegenwart einer Lewissäure mit einem Benzol der Formel III

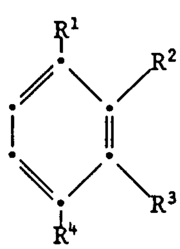

(III)

umsetzt, wobei $R^1$ bis $R^8$ die zuvor angegebenen Bedeutungen haben, und gegebenenfalls Verbindungen der Formel I, worin mindestens eines von $R^1$ bis $R^4$, $R^6$ und $R^7$-$NO_2$ oder Halogen bedeuten, mit einer nukleophilen Verbindung substituiert. Halogen ist bevorzugt -Br, -Cl und besonders -F. Für die nukleophile Substitution geeignete Verbindungen sind besonders solche der Formel ($R^1$ bis $R^4$, $R^6$ und $R^7$}X-H, worin X -O-, -S-, -SO- oder $-SO_2$-sind, $H-NR^9R^{10}$, Malonsäureester oder -nitrile und Phenylessigsäurenitril. Die Verbindungen können in Form ihrer Alkalisalze, z.B. Li-, Na- oder K-Salze verwendet werden. Ebenso ist es möglich, die nukleophile Substitution in Gegenwart von Basen, wie z.B. Alkalilaugen oder -carbonaten

durchzuführen.

Die Verbindungen der Formel I können auch hergestellt werden, indem man in einer Diels-Alder-Reaktion eine Verbindung der Formel IV

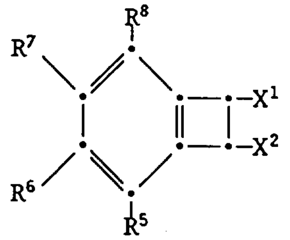

(IV)

worin $R^5$, $R^6$, $R^7$ und $R^8$ die zuvor angegebenen Bedeutungen haben und $X^1$ und $X^2$ unabhängig voneinander für -Cl, -Br - oder -J stehen, unter Abspaltung von $HX^1$ und $HX^2$ mit einer Verbindung der Formel V

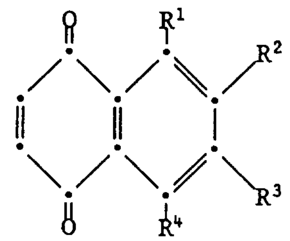

(V),

worin $R^1$, $R^2$, $R^3$ und $R^4$ die zuvor angegebenen Bedeutungen haben, umsetzt. In den Substituenten $R^1$ bis $R^4$, $R^6$ und $R^7$ ist p gleich 1.

Die Reaktion wird vorteilhaft bei Temperaturen von 50 bis 250 °C, bevorzugt 80 bis 200 °C durchgeführt. Zweckmässig wird ein inertes Lösungsmittel verwendet, zum Beispiel polare aprotische Lösungsmittel. Einige Beispiele sind aromatische Kohlenwasserstoffe (Benzol, Toluol, Xylol, Chlorbenzol und Dichlorbenzol), Nitrile (Acetonitril), Ether (Dibutylether, Dioxan, Tetrahydrofuran, Ethylenglykol- oder Diethylenglykoldimethylether). Die Isolierung und Reinigung kann nach üblichen Methoden erfolgen, z.B. Kristallisation, Sublimation oder Chromatographie.

Verbindungen der Formel IV sind teilweise bekannt (siehe z.B. H.P. Cava et al., J. Am. Chem. Soc., S. 1701 (1957) und J.W. Barton et al., J. Chem. Soc. Perkin Trans. 1, S. 967-971 (1986)), bzw. nach analogen Verfahren herstellbar. Die für die Herstellung benötigten substituierten 1,2-Bis(dichlor- oder -dibrommethyl)-benzole sind ebenfalls teilweise bekannt oder durch übliche elektrophile oder nukleophile Substitutionsreaktionen entsprechender o-Dimethylbenzole, und deren anschliessenden Chlorierung oder Bromierung mit z.B. N-Chlor- oder N-Bromsuccinimid erhältlich.

Die 1,4-Naphthochinone der Formel V sind bekannt und z.B. durch nukleophile Substitution von gegebenenfalls geschützten und substituierten Halogen-oder Nitro-1,4-naphthochinonen mit z.B. den zuvor beschriebenen Verbindungen in Gegenwart von Alkalimetallverbindungen ($K_2CO_3$, $Cs_2CO_3$, KOH, NaOH, $NaNH_2$, $NaOCH_3$, $NaOC_2H_5$) oder mit Alkalimetall-Verbindungen z.B. von Li, K, Na, Rb oder Cs, erhältlich. Halogen-und Nitro-naphthochinone sind z.B. in Houben-Weyl, Chinone I, Band 7/3b (1977) beschrieben. Die Naphthochinone der Formel V können auch in bekannter Weise durch elektrophile oder nukleophile Substitution von gegebenenfalls substituierten Naphthalinen, Dihydro- oder Tetrahydronaphthalinen und anschliessende Umwandlung in die substituierten 1,4-Naphthochinone hergestellt werden.

Die Verbindungen der Formel I können auch hergestellt werden, indem man 1,2-Bis(dihalogenmethyl)-benzole der Formel

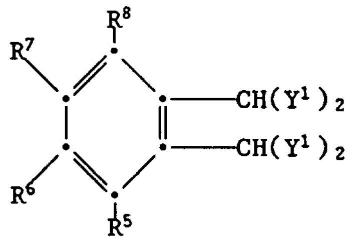

,

worin $Y^1$ für Cl, Br oder I steht, in Gegenwart von NaI in einem organischen Lösungsmittel mit einer Verbindung der Formel V umsetzt. Diese Methode ist von J.W. McOmie in Synthesis, S. 416-417 (1973) beschrieben.

Verbindungen der Formel I können auch erhalten werden, indem man Anthracen-1,4-chinone der Formel

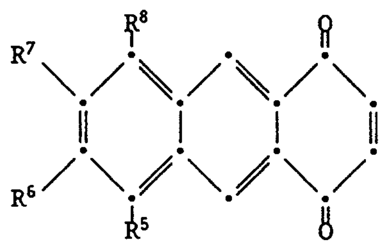

mit einem α-Pyron der Formel VI

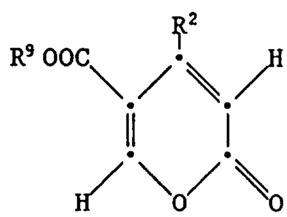

(VI),

oder einem Butadien der Formel VII

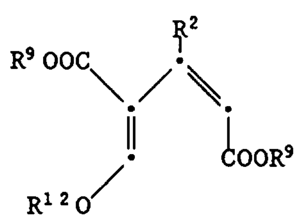

(VII)

umsetzt, wobei $R^{11}$ $C_1$-$C_6$-Alkyl ist, $R^9$ die zuvor angegebene Bedeutung hat und bevorzugt $C_1$-$C_6$-Alkyl ist. Diese Methode und die Herstellung von α-Pyronen ist in der US-PS 4,617,151 und der EP-A-0 195 743 beschrieben.

Verbindungen der Formeln VI und VII sind z.B. folgendermassen erhältlich;
wobei $X^3$ ein Alkalimetall bedeutet:

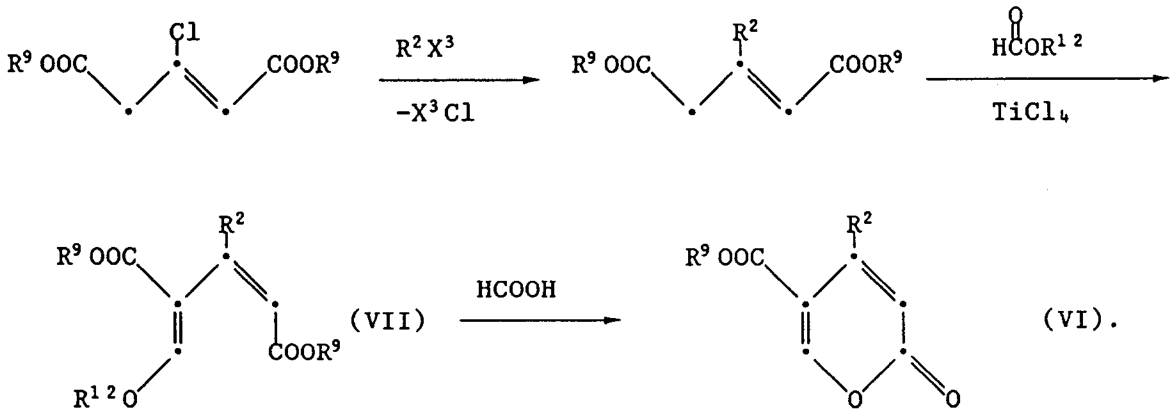


(VII)

(VI).

Wenn $R^1$ bis $R^4$, $R^6$ und $R^7$ einen Polyoxaalkylenrest bedeuten, so erhält man solche Verbindungen auch durch Umsetzung von Verbindungen der Formel I mit $R^1$ bis $R^4$, $R^6$ und $R^7$ gleich Hydroxyalkyl mit Epoxiden. Ferner ist es möglich die Reste $R^1$ bis $R^4$, $R^6$ und $R^7$ durch klassische Reaktionen abzuwandeln, wie z.B. Hydrolyse, Ver- oder Umesterung, Amidierung, Oxidation oder Reduktion. Carbonsäureester können in bekannter Weise mit $HF/SF_4$ in die Trifluormethylderivate umgewandelt werden.

Die Verbindungen der Formel I sind im allgemeinen kristallin und zeichnen sich durch eine hohe thermische Stabilität aus. Verbindungen mit Oxy-, Sulfinyl- oder Alkylsubstituenten können sehr gut in härtbaren Zusammensetzungen gelöst werden, gegebenenfalls zusammen mit einem Lösungsmittel.

Sie eignen sich alleine oder zusammen mit H-Donoren wie z-B. tertiären Aminen oder Alkoholen oder Phenylessigsäurederivaten als wirksame Photoinitiatoren bzw. Sensibilisatoren für die lichtinduzierte Polymerisation bzw. Dimerisation ethylenisch ungesättigter Verbindungen. Sie zeichnen sich hierbei durch eine gute Lichtempfindlichkeit und Wirksamkeit über einen Wellenlängenbereich von ca. 200 nm (UV-Licht) bis etwa 600 nm aus.

Die Eigenschaften der erfindungsgemässen Verbindungen, z.B. Löslichkeit, Schmelzpunkt und Absorptionsbereich lassen sich durch die Wahl von Substituenten gezielt beeinflussen.

Ein weiterer Gegenstand vorliegender Erfindung ist eine durch Strahlung polymerisierbare Zusammensetzung, enthaltend (a) mindestens eine nichtflüchtige, monomere, oligomere oder polymere Verbindung mit mindestens einer photopolymerisierbaren oder photodimerisierbaren ethylenisch ungesättigten Doppelbindung und (b) mindestens eine Verbindung der Formel Ia als Photoinitiator oder Sensibilisator,

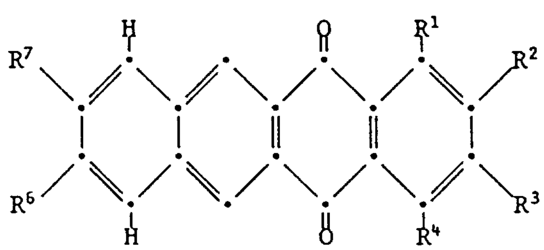

(Ia),

worin mindestens eines von $R^1$ bis $R^4$, $R^6$ und $R^7$ einen Substituenten darstellen, $R^1$ und $R^4$ unabhängig voneinander H, $C_1$-$C_{18}$-Alkoxy, $-O(C_mH_{2m}-O)_nR^{11}$ mit m gleich 2 bis 4, n gleich 1 bis 20 und $R^{11}$ gleich H oder $C_1$-$C_{12}$-Alkyl, $R^2$, $R^3$, $R^6$ und $R^7$ unabhängig voneinander H $C_1$-$C_{18}$-Alkoxy, $C_1$-$C_{18}$-Alkyl-SO-, $-O(C_mH_{2m}-O)_nR^{11}$, $-COO(C_1$-$C_{18}$-Alkyl), $-COO(C_mH_{2m}-O)_nR^{11}$ oder mit 1 oder 2 $-COO(C_1$-$C_{18}$-Alkyl) oder $-COO(C_mH_{2m}-O)_nR^{11}$ substituiertes $C_1$-$C_6$-Alkyl, Benzyl oder $C_1$-$C_6$-Alkoxy darstellen, wobei $R^{11}$, m und n die zuvor angegebenen Bedeutungen haben.

Die Zusammensetzungen können weitere von (b) verschiedene Photoinitiatoren oder Sensibilisatoren enthalten.

Die Zusatzmenge an erfindungsgemässen Verbindungen richtet sich im wesentlichen nach wirtschaftlichen Gesichtspunkten, deren Löslichkeiten und nach der gewünschten Empfindlichkeit. Im allgemeinen werden 0,01 bis 20, vorzugsweise 0,05-10 und besonders 0,1 bis 5 Gew.% verwendet, bezogen auf die Komponente (a).

Als Komponente (a) kommen solche ethylenisch ungesättigten monomeren, oligomeren und polymeren Verbindungen in Frage, die durch Photopolymerisation zu höhermolekularen Produkten reagieren und hierbei ihre Löslichkeit verändern.

Besonders geeignet sind z.B. Ester von ethylenisch ungesättigten Carbonsäuren und Polyolen oder Polyepoxiden, und Polymere mit ethylenisch ungesättigten Gruppen in der Kette oder in Seitengruppen, wie z.B. ungesättigte Polyester, Polyamide und Polyurethane und Copolymere hiervon, Polybutadien und Butadien-Copolymere, Polyisopren und Isopren-Copolymere, Polymere und Copolymere mit (Meth)-Acrylgruppen in Seitenketten, sowie Mischungen von einem oder mehreren solcher Polymerer.

Beispiele für ungesättigte Carbonsäuren sind Acrylsäure, Methacrylsäure, Crotonsäure, Itaconsäure, Zimtsäure, ungesättigte Fettsäuren wie Linolensäure oder Oelsäure. Bevorzugt sind Acryl- und Methacrylsäure.

Als Polyole sind aromatische und besonders aliphatische und cycloaliphatische Polyole geeignet. Beispiele für aromatische Polyole sind Hydrochinon, 4,4'-Dihydroxydiphenyl, 2,2-Di(4-hydroxyphenyl)-propan, sowie Novolake und Resole. Beispiele für Polyepoxide sind solche auf der Basis der genannten Polyole, besonders der aromatischen Polyole und Epichlorhydrin. Ferner sind auch Polymere oder Copolymere, die Hydroxylgruppen in der Polymerkette oder in Seitengruppen enthalten, wie z.B. Polyvinylalkohol und Copolymere davon oder Polymethacrylsäurehydroxyalkylester oder Copolymere davon, als Polyole geeignet. Weitere geeignete Polyole sind Oligoester mit Hydroxylendgruppen.

Beispiele für aliphatische und cycloaliphatische Polyole sind Alkylendiole mit bevorzugt 2 bis 12 C-Atomen, wie Ethylenglykol, 1,2- oder 1,3-Propandiol, 1,2-, 1,3 oder 1,4-Butandiol, Pentandiol, Hexandiol, Octandiol, Dodecandiol, Diethylenglykol, Triethylenglykol, Polyethylenglykole mit Molekulargewichten von bevorzugt 200 bis 1500, 1,3-Cyclopentandiol, 1,2-, 1,3- oder 1,4-Cyclohexandiol, 1,4-Dihydroxymethylcyclohexan, Glycerin, Tris-(β-hydroxyethyl)amin, Trimethylolethan, Trimethylolpropan, Pentaerythrit, Dipentaerythrit und Sorbit.

Die Polyole können teilweise oder vollständig mit einer oder verschiedenen ungesättigten Carbonsäuren verestert sein, wobei in Teilestern die freien Hydroxylgruppen modifiziert, z.B. verethert oder mit anderen Carbonsäuren verestert sein können.

Beispiele für Ester sind:

Trimethylolpropantriacrylat, Trimethylolethantriacrylat, Trimethylolpropantrimethacrylat, Trimethylolethantrimethacrylat, Tetramethylenglykoldimethacrylat, Triethylenglykoldimethacrylat, Tetraethylenglykoldiacrylat, Pentaerythritdiacrylat, Pentaerythrittriacrylat, Pentaerythrittetraacrylat, Dipentaerythritdiacrylat, Dipentaerythrittriacrylat, Dipentaerythrittetraacrylat, Dipentaerythritpentaacrylat, Dipentaerythrithexaacrylat, Tripentaerythritoctaacrylat, Pentaerythritdimethacrylat, Pentaerythrittrimethacrylat, Dipentaerythritdimethacrylat, Dipentaerythrittetramethacrylat, Tripentaerythritoctamethacrylat, Pentaerythritdiitaconat, Dipentaerythrittrisitaconat, Dipentaerythritpentaitaconat, Dipentaerythrithexaitaconat, Ethylenglykoldimethacrylat, 1,3-Butandioldiacrylat, 1,3-Butandioldimethacrylat, 1,4-Butandioldiitaconat, Sorbittriacrylat, Sorbittetraacrylat, Pentaerythrit-modifiziert-triacrylat, Sorbittetramethacrylat, Sorbitpentaacrylat, Sorbithexaacrylat, Oligoesteracrylate und -methacrylate, Glyzerindi- und -triacrylat, 1,4-Cyclohexandiacrylat, Bisacrylate und Bismethacrylate von Polyethylenglykol mit Molekulargewicht von 200-1500, oder Gemische davon.

Als Komponente (a) sind auch die Amide gleicher oder verschiedener ungesättigter Carbonsäuren von aromatischen, cycloaliphatischen und aliphatischen Polyaminen mit bevorzugt 2 bis 6, besonders 2 bis 4 Aminogruppen geeignet. Beispiele für solche Polyamine sind Ethylendiamin, 1,2-oder 1,3-Propylendiamin, 1,2-, 1,3- oder 1,4-Butylendiamin, 1,5-Pentylendiamin, 1,6-Hexylendiamin, Octylendiamin, Dodecylendiamin, 1,4-Diaminocyclohexan, Isophorondiamin, Phenylendiamin, Bisphenylendiamin, Di-β-aminoethylether, Diethylentriamin, Triethylentetramin, Di-(β-aminoethoxy)- oder Di(β-aminopropoxy)ethan. Weitere geeignete Polyamine sind Polymere und Copolymere mit Aminogruppen in der Seitenkette und Oligoamide mit Aminoendgruppen.

Beispiele für solche ungesättigten Amide sind: Methylen-bis-acrylamid, 1,6-Hexamethylen-bis-acrylamid, Diethylentriamin-tris-methacrylamid, Bis(methacrylamidopropoxy)-ethan, β-Methacrylamidoethylmethacrylat,N[(β-Hydroxyethoxy)ethyl]-acrylamid.

Geeignete ungesättigte Polyester und Polyamide leiten sich z.B. von Maleinsäure und Diolen oder Diaminen ab. Die Maleinsäure kann teilweise durch andere Dicarbonsäuren ersetzt sein. Sie können zusammen mit ethylenisch ungesättigten Comonomeren, z.B. Styrol, eingesetzt werden. Die Polyester und Polyamide können sich auch von Dicarbonsäuren und ethylenisch ungesättigten Diolen oder Diaminen ableiten, besonders von längerkettigen mit z.B. 6 bis 20 C-Atomen. Beispiele für Polyurethane sind solche, die aus gesättigten oder ungesättigten Diisocyanaten und ungesättigten bzw. gesättigten Diolen aufgebaut sind.

Polybutadien und Polyisopren und Copolymere davon sind bekannt. Geeignete Comonomere sind z.B. Polyolefine wie Ethylen, Propen, Buten, Hexen, (Meth)Acrylate, Acrylnitril, Styrol oder Vinylchlorid. Polymere mit (Meth)Acrylatgruppen in der Seitenkette sind ebenfalls bekannt. Es kann sich z.B. um Umsetzungsprodukte von Epoxidharzen auf Novolakbasis mit (Meth)Acrylsäure handeln, um Homo- oder Copolymere des Polyvinylalkohols oder deren Hydroxyalkylderivaten, die mit (Meth)Acrylsäure verestert sind, oder um Homo- und Copolymere von (Meth)Acrylaten, die mit Hydroxyalkyl(meth)acrylaten verestert sind.

Die photopolymerisierbaren Verbindungen können alleine oder in beliebigen Mischungen eingesetzt werden. Bevorzugt werden Gemische von Polyol(Meth)Acrylatenverwendet.

Geeignete dimerisierbare Verbindungen sind solche, die z.B. Zimtsäurereste, Dimethylmaleinimidylreste oder

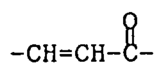 oder 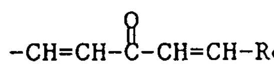-Reste

enthalten.
Diese Reste sind im allgemeinen an Oligomere oder Polymere gebunden, z.B. in der Polymerkette oder als Seitengruppen. Polymere mit Dimethylmaleinimidylgruppen sind z.B. in der DE-A-2626795 beschrieben.
Epoxidharze mit

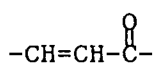 oder 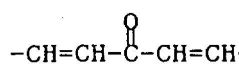-Gruppen

sind z.B. in der DE-A-2342407 beschrieben.

Den erfindungsgemässen Zusammensetzungen können auch Bindemittel zugesetzt werden, was besonders zweckmässig ist, wenn es sich bei den photopolymerisierbaren bzw. photodimerisierbaren Verbindungen um flüssige oder viskose Substanzen handelt. Die Menge des Bindemittels kann z.B. 5-95, vorzugsweise 10-90 und besonders 50-90 Gew.% betragen, bezogen auf die gesamte Zusammensetzung. Die Wahl des Bindemittels erfolgt je nach dem Anwendungsgebiet und hierfür geforderter Eigenschaften wie Entwikkelbarkeit in wässrigen und organischen Lösungsmittelsystemen, Adhäsion auf Substraten und Sauerstoffempfindlichkeit.

Geeignete Bindemittel sind z.B. Polymere mit einem Molekulargewicht von etwa 5000-2 000 000, bevorzugt 10 000 bis 1 000 000. Beispiele sind: Homo- und copolymere Acrylate und Methacrylate, z.B. Copolymere aus Methylmethacrylat/Ethylacrylat/Methacrylsäure, Poly(methacrylsäurealkylester), Poly(acrylsäurealkylester); Celluloseester und -ether wie Celluloseacetat, Celluloseacetatbutyrat, Methylcellulose, Ethylcellulose; Polyvinylbutyral, Polyvinylformal, cyclisierter Kautschuk, Polyether wie Polyethylenoxid, Polypropylenoxid, Polytetrahydrofuran; Polystyrol, Polycarbonat, Polyurethan, chlorierte Polyolefine, Polyvinylchlorid, Copolymere aus Vinylchlorid/Vinylidenchlorid, Copolymere von Vinylidenchlorid mit Acrylnitril, Methylmethacrylat und Vinylacetat, Polyvinylacetat, Copoly(ethylen/vinylacetat), Polyamide wie Polycaprolactam und Poly(hexamethylenadipamid), Polyester wie Poly(äthylenglykolterephthalat) und Poly(hexamethylenglykolsuccinat).

Die erfindungsgemässen Zusammensetzungen eignen sich als Beschichtungsmittel für Substrate aller Art, z.B. Holz, Papier, Keramik, Kunststoffe, wie Polyester und Celluloseacetatfilme, und Metalle, wie Kupfer und Aluminium, bei denen durch Photopolymerisation eine Schutzschicht oder eine photographische Abbildung aufgebracht werden soll. Ein weiterer Gegenstand vorliegender Erfindung sind die beschichteten Substrate und ein Verfahren zum Aufbringen photographischer Abbildungen auf den Substraten. Die beschichteten Substrate können auch als Aufzeichnungsmaterial für Hologramme (Volumen-Phasen-Diagramm) verwendet werden, wobei vorteilhaft ist, dass für diesen Zweck keine Nassentwicklung notwendig ist.

Die Beschichtung der Substrate kann erfolgen, indem man eine flüssige Zusammensetzung, eine Lösung oder Suspension auf das Substrat aufbringt. Flüssige Zusammensetzungen ohne Lösungsmittel sind bevorzugt.

Die Wahl des Lösungsmittels und die Konzentration richtet sich hauptsächlich nach der Art der Zusammensetzung und nach dem Beschichtungsverfahren. Die Zusammensetzung wird mittels bekannter Beschichtungsverfahren auf ein Substrat gleichförmig aufgebracht, z.B. durch Tauchen, Rakelbeschichtung, Vorhanggiessverfahren, Elektrophorese, Aufpinseln, Sprayen oder Reverseroll-Beschichtung. Die Auftragsmenge (Schichtdicke) und Art des Substrates (Schichtträger) sind abhängig vom gewünschten Applikationsgebiet. Als Schichtträger für photographische Informationsaufzeichnung dienen z.B. Folien aus Polyester, Celluloseacetat oder mit Kunststoff beschichtete Papiere; für Offsetdruckformen speziell behandeltes Aluminium und für die Herstellung gedruckter Schaltungen kupferkaschierte Laminate. Die Schichtdicken für photographische Materialien und Offsetdruckformen betragen im allgemeinen ca. 0,5 bis ca. 10 µm; für gedruckte Schaltungen im allgemeinen 1 bis ca. 100 µm. Bei Mitverwendung von Lösungsmitteln werden diese nach dem Beschichten entfernt.

Photohärtbare Zusammensetzungen, wie sie für die verschiedenen Zwecke verwendet werden, enthalten meist ausser den photopolymerisierbaren Verbindungen und den Photoinitiatoren eine Reihe sonstiger Zusätze. So ist es vielfach üblich, thermische Inhibitoren zuzusetzen, die vor allem während der Herstellung der Zusammensetzungen durch Mischen der Komponenten vor einer vorzeitigen Polymerisation schützen sollen. Hierzu werden beispielsweise Hydrochinon, Hydrochinonderivate, p-Methoxyphenol, β-Naphthole oder sterisch gehinderte Phenole wie z.B. 2,6-Di(tert-butyl)-p-kresol verwendet. Weiter können geringe Mengen von UV-Absorbern zugesetzt werden, wie z.B. solche vom Benztriazol-, Benzophenon- oder Oxalanilid-Typ. Ebenso lassen sich Lichtschutzmittel vom Typus sterisch gehinderter Amine (HALS) zusetzen.

Zur Erhöhung der Dunkellagerstabilität können Kupferverbindungen, wie Kupfernaphthenat, -stearat, oder -octoat, Phosphorverbindungen, wie Triphenylphosphin, Tributylphosphin, Triethylphosphit, Triphenylphosphit oder Tribenzylphosphit, quaternäre Ammoniumverbindungen, wie Tetramethylammoniumchlorid oder Trimethyl-benzylammoniumchlorid oder Hydroxylaminderivate, wie z.B. N-Diethylhydroxylamin, zugesetzt werden.

Um die inhibierende Wirkung des Luftsauerstoffs auszuschliessen, setzt man photohärtbaren Gemischen häufig Paraffin oder ähnliche wachsartige Stoffe zu. Diese schwimmen bei Beginn der Polymerisation wegen mangelnder Löslichkeit im Polymeren aus und bilden eine transparente Oberflächenschicht, die den Zutritt von Luft verhindert.

Weitere übliche Zusätze sind Photosensibilisatoren, welche in bestimmten Wellenlängen absorbieren und die absorbierte Energie an den Initiatoren weitergeben oder selbst als zusätzlicher Initiator fungieren. Beispiele hierfür sind vor allem Thioxanthon-, Anthracen-, Anthrachinon- und Cumarinderivate.

Weitere übliche Zusätze sind Beschleuniger vom Amin-Typ, die vor allem in pigmentierten Zubereitungen von Bedeutung sind, da sie als Kettenüberträger wirken. Beispiele hierfür sind N-Methyldiethanolamin, Triethylamin, p-Dimethylaminobenzoesäureethylester oder Michler's Keton. Die Wirkung der Amine kann verstärkt werden durch den Zusatz von aromatischen Ketonen vom Benzophenontyp.

Weitere übliche Zusätze sind z.B. Füllstoffe, Pigmente, Farbstoffe, Haft-, Netz- und Verlaufmittel.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Insbesondere für den Siebdruck sind UV-härtbare Druckfarben von Bedeutung.

Gut geeignet sind die erfindungsgemässen photohärtbaren Zusammensetzungen auch zur Herstellung von Druckplatten, insbesondere Flexodruckplatten. Hierbei werden z.B. Gemische von löslichen linearen Polyamiden oder von Styrol-Butadien-Kautschuk mit photopolymerisierbaren Monomeren, beispielsweise Acrylamiden oder Acrylaten, und einem Photoinitiator verwendet. Filme und Platten aus diesen Systemen werden über das Negativ (oder Positiv) der Druckvorlage belichtet und die ungehärteten Anteile anschliessend mit einem Lösungsmittel eluiert.

Ein weiteres Einsatzgebiet der Photohärtung ist die Metallbeschichtung, beispielsweise bei der Lackierung von Blechen für Tuben, Dosen oder Flaschenverschlüsse, sowie die Photohärtung von Kunststoffbeschichtungen, beispielsweise von Fussboden- oder Wandbelägen auf PVC-Basis.

Beispiele für die Photohärtung von Papierbeschichtungen sind die farblose Lackierung von Etiketten, Schallplatten-Hüllen oder Buchumschlägen.

Wichtig ist auch die Verwendung der photohärtbaren Zusammensetzungen für Abbildungsverfahren und zur optischen Herstellung von Informationsträgern. Hierbei wird die auf dem Träger aufgebrachte Schicht (nass oder trocken) durch eine Photomaske mit kurzwelligem Licht bestrahlt und die unbelichteten Stellen der Schicht durch Behandlung mit einem Lösungsmittel (= Entwickler) entfernt. Die belichteten Stellen sind vernetztpolymer und dadurch unlöslich und bleiben auf dem Träger stehen. Bei entsprechender Anfärbung entstehen sichtbare Bilder. Ist der Träger eine metallisierte Schicht, so kann das Metall nach dem Belichten und Entwickeln an den unbelichteten Stellen weggeätzt oder durch Galvanisieren verstärkt werden. Auf diese Weise lassen sich gedruckte Schaltungen herstellen. Die erfindungsgemässe Zusammensetzung kann auch als Photoresist verwendet werden.

Zur Belichtung eignen sich Lichtquellen mit hohem Anteil an kurzwelligem Licht. Hierfür stehen heute entsprechende technische Vorrichtungen und verschiedene Lampenarten zur Verfügung. Beispiele sind

Kohlelichtbogenlampen, Xenonlichtbogenlampen, Quecksilberdampflampen, Metall-Halogenlampen, Fluoreszenzlampen, Argonlampen oder photographische Flutlichtlampen. Neuerdings werden auch Laserlichtquellen verwendet. Diese haben den Vorteil, dass keine Photomasken notwendig sind; der gesteuerte Laserstrahl schreibt direkt auf die photohärtbare Schicht.

Weitere Gegenstände der Erfindung sind somit

a) ein beschichtetes Substrat, das auf mindestens einer Oberfläche mit einer erfindungsgemässen Zusammensetzung beschichtet ist;

b) ein Verfahren zur photographischen Herstellung von Reliefabbildungen oder Beschichtungen, dadurch gekennzeichnet, dass man ein beschichtetes Substrat bildmässig oder flächenmässig belichtet und unbelichtete Anteile danach mit einem Lösungsmittel entfernt;

c) die Verwendung von Verbindungen der Formel Ia als Initiatoren und Sensibilisatoren für die Photopolymerisation oder Photodimerisation von nichtflüchtigen monomeren, oligomeren oder polymeren Verbindungen mit mindestens einer photopolymerisierbaren oder photodimerisierbaren ethylenisch ungesättigten Doppelbindung; und

d) die Verwendung einer erfindungsgemässen Zusammensetzung zur Herstellung von Lacken, Druckfarben, Druckplatten, Resistmaterialien sowie als Bildaufzeichnungsmaterial und Beschichtungsmittel.

Die Verbindungen der Formel I sind auch wertvolle Zwischenprodukte zur Herstellung von substituierten Tetrathio- bzw. Tetraselenotetracenen (vgl. US-PS 4 617 151). Aus solchen chalkogenierten Tetracenen können mit Elektronenacceptoren elektrisch leitende Charge-Transferkomplexe (CT-Komplexe) hergestellt werden. Mit deren funktionellen Substituenten kann man sie an Polymere binden, z.B. als Seitengruppen in Polymere einbauen (vgl. US-PS 4,617,151). Die CT-Komplexe eignen sich auch zur Herstellung z.B. von antistatischen Beschichtungen photographischer Filmelemente, Magnetbänder, elektrophotographischer Filmelemente und elektronischer Komponenten (siehe US-PS 3,634,336). Die chalkogenierten Tetracene weisen ferner elektrochrome Eigenschaften auf; sie können für elektrochrome Displays verwendet werden. Sie eignen sich auch als laser-optische Datenspeicher [Nach. Chem. Techn. Lab. 35, S. 255 ff (1987)] und als Anodenmaterial in organischen Solid-State-Batterien (EP-A-0 090 598). CT-Komplexe von substituierten Tetrathio- oder Tetraselenotetracenen können zur Erzielung antistatischer Eigenschaften auch in thermoplastische, duroplastische oder elastomere Polymere eingearbeitet werden. Hierzu werden zweckmässig z.B. die substituierten Tetrathio- bzw. Tetraselenotetracene zusammen mit einem löslichen Polymer oder einer Vorstufe davon und einem Elektronenacceptor, z.B. einem Halogen bildenden Mittel (organische halogenierte Verbindungen wie z.B. Bromoform, Trichlorbrommethan, Tetrabrommethan, Hexachlorpropan, Perchlorbutadien, 1,3-oder 1,4-Dichlor-2-buten, 1,4-Bis(trichloromethyl)benzol, Iodacetonitril, Iodoform, Tetrachlorethylen, Perchlorcyclobutadien, N-Chlor-, -brom oder -iodsuccinimid) gegebenenfalls zusammen mit einem weiteren inerten Lösungsmittel gelöst und das überschüssige Halogen bildende Mittel und das Lösungsmittel bei höherer Temperatur verdampft. Die gebildete Zusammensetzung enthält im Polymer ein Netzwerk nadelförmiger Kristalle des CT-Komplexes, wenn das chalkogenierte Tetracen unsubstituiert ist oder kleine Substituenten (z.B. F, $CH_3$, $CF_3$) enthält. Solche Zusammensetzungen weisen eine hohe elektrische Leitfähigkeit auf. Diese kann noch verbessert werden, wenn man ein aus den Verbindungen der Formel I hergestelltes substituiertes Tetrathio-oder Tetraselenotetracen mitverwendet, das kein solches Netzwerk bildet und das in der Polymermatrix in feiner Verteilung vorliegt, da solche substituierten Tetrathio- oder Tetraselenotetracene keine oder nur eine geringe Kristallisationstendenz im Polymer besitzen. Naphthacen-5,12-dione können ferner auch in elektrochromen Displayelementen verwendet werden (JP-OS 61-43680).

Die Verbindungen der Formel I können auch zur stromlosen Metallabscheidung auf der Oberfläche von Hydroxyalkylgruppen enthaltenden Polymeren, z.B. Copolymeren von Methacrylaten mit Alkyl- und Hydroxyalkylestergruppen, oder Epoxiharzen, die z.B. mit Aminoalkoholen gehärtet sind. Hierzu vermischt man Verbindungen der Formel I mit den Polymeren, z.B. in einer Menge von 0,1 bis 10 Gew.-%, bezogen auf das Polymer, belichtet gegebenenfalls unter einer Negativvorlage und behandelt mit einem Metallierungsbad, das vorzugsweise ein Kupfersalz enthält.

Die nachfolgenden Beispiele erläutern die Erfindung näher.

<u>A) Herstellungsbeispiele:</u>

<u>Beispiele 1-17</u>: <u>2-n-Octyloxy-naphthacen-5,12-dion:</u>

20 g (72,4 mMol) 2-Fluor-naphthacen-5,12-dion, 94,3 g 1-Octanol, 30,01 g (217,2 mMol) wasserfreies Kaliumcarbonat und 200 ml Dimethylsulfoxid (DMSO) werden während 20 Std. bei 100 °C Badtemperatur gerührt. Das Reaktionsgemisch wird abgekühlt, mit Toluol/verdünnter Salzsäure versetzt, die organische Phase abgetrennt, zweimal mit Wasser gewaschen, mit Natriumsulfat getrocknet und eingedampft. Der

Rückstand wird mit Pentan gewaschen und aus Cyclohexan umkristallisiert. Ausbeute: 22,9 g (82 %), Schmelzpunkt 127-129 ° C.

Analog werden die in Tabelle 1 beschriebenen Verbindungen unter Verwendung der entsprechenden Alkohole hergestellt.

Tabelle 1

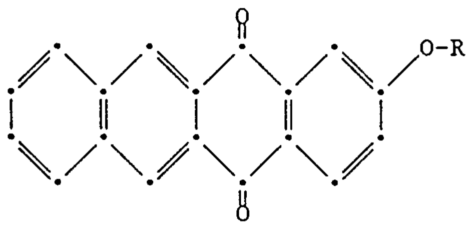

| Beispiel | R | Reaktionszeit (Stunden) | Badtemperatur (°C) | Ausbeute (%) | Smp. (°C) |
|---|---|---|---|---|---|
| 2 | 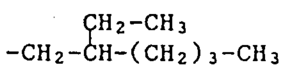 | 15 | 130 | 41 | 85-90 |
| 3 | $-(CH_2)_2-CH(CH_3)-(CH_2)_3-CH(CH_3)_2$ | 16 | 140 | 12 | 102-104 |
| 4 | $-CH_2-C{\equiv}CH$ | 23 | 60 | 84 | 160-163 |
| 5 | -H*) | 18 | 130 | 61 | >250 |
| 6 | $-CH_3$ | 21 | 60 | 94 | 237-239 |
| 7 | $-CH_2-CH_3$ | 5 | 100 | 95 | 198-200 |
| 8 | $-CH(CH_3)_2$ | 18 | 127 | 34 | 112-114 |
| 9 | 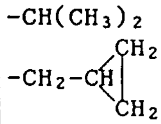 | 18 | 110 | 46 | 200-203 |
| 10 | $-n-C_{18}H_{37}$ | 26 | 120 | 79 | 58-103 |
| 11 | 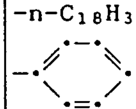 | 1 | 100 | 86 | 217-219 |
| 12 | 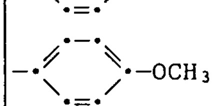 | 1/4 | 100 | 83 | 222-223 |
| 13 | 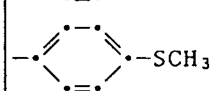 | 1/3 | 105 | 84 | 203-205 |
| 14 | $-(CH_2)_2-O-(CH_2)_2-OH$ | 3 | 100 | 82 | 130-132 |
| 15 | $-CH_2-CH(OH)-CH_2-OH$ | 3 | 100 | 76 | 187-191 |
| 16 | $-(CH_2-CH_2-O)-CH_3$ | 8 | 100 | 72 | 90-93 |
| 17 | $-(CH_2-CH_2-O)_{12}-CH_3$ | 8 | 100 | 60 | (teilkristallin) |

*) Einleitung von $H_2S$

Beispiele 18-23:

2-(2'-Hydroxyethoxy)-naphthacen-5,12-dion

27,6 g (0,1 mol) 2-Fluor-5,12-naphthacenchinon, 32,5 g (0,1 mol) $Cs_2CO_3$ und 300 ml Ethylenglycol werden in einem Sulfierkolben unter Stickstoff vorgelegt. Nach Erwärmen auf 125 °C wird $3^1/_4$ h gerührt. Danach wird das Reaktionsgemisch in 3000 ml salzsaures Wasser gegossen, das ausgefallene Produkt abfiltriert und mehrmals mit Wasser gewaschen. Nach dem Trocknen im Vakuum bei 80 °C werden 30,3 g (97,1 %) reines Produkt erhalten, Smp. 208,8 °C. Analog wird in den Beispielen 19-23 verfahren (s. Tabelle 2).

Beispiele 24-27:

2-(3-Butenoxy)-naphthacen-5,12-dion

10 g (0,036 mol) 2-Fluor-5,12-naphthacenchinon, 26 g (0,028 mol) $Cs_2CO_3$ und 10,41 g (0,14 mol) 3-Butenol werden in 200 ml DMF unter Stickstoff vorgelegt. Die Reaktionsmischung wird auf 125 °C erwärmt und $4^1/_2$ h gerührt. Nach dem Fällen in 4000 ml salzsaurem Wasser wird abfilteriert (Rohausbeute 94 %). Nach Chromatographie an Silicagel ($CH_2Cl_2$) werden 63 % reines Produkt erhalten; Smp. 149 °C. Analog wird in den Beispielen 25-27 verfahren (s. Tabelle 2).

Tabelle 2

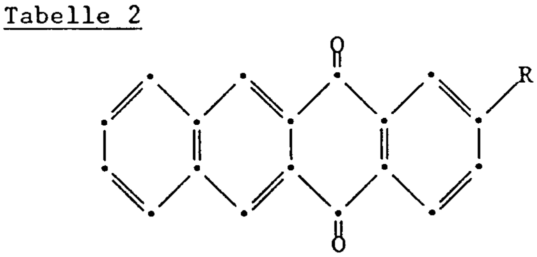

| Beispiel | R | Smp. (°C) | Reaktionszeit (Stunden) | Ausbeute (%) | Lösungsmittel | Reaktionstemperatur (°C) |
|---|---|---|---|---|---|---|
| 18 | $O-(CH_2)_2-OH$ | 208 | $3\ ^1/_4$ | 97 | i.S. | 125 |
| 19 | $O-(CH_2)_4-OH$ | 190 | $7\ ^1/_2$ | 70 | i.S. | 120 |
| 20 | $O-(CH_2)_6-OH$ | 153 | $1\ ^1/_4$ | 70 | DMSO | 120 |
| 21 | $O-(CH_2)_{10}-OH$ | 113 | $^3/_4$ | 73 | i.S. | 125 |
| 22 | $O-CH_2-(CF_2)_3-CH_2-OH$ | 167 | $3\ ^1/_4$ | 79 | DMF | 90 |
| 23 | 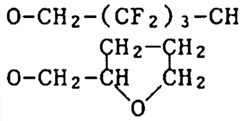 | 144-147 | $19\ ^1/_2$ | 75 | DMF | 110 |
| 24 | $O-CH_2-CH=CH_2$ | 164 | $5\ ^1/_4$ | 91 | i.S. | 95 |
| 25 | $O-(CH_2)_2-CH=CH_2$ | 152 | $4\ ^1/_2$ | 84 | DMF | 130 |
| 26 | $O-(CH_2)_4-CH=CH_2$ | 125 | $5\ ^1/_2$ | 78 | DMF | 130 |
| 27 | $O-(CH_2)_9-CH=CH_2$ | 98 | $5\ ^1/_2$ | 65 | i.S. | 130 |

i.S.: Alkohol ist gleich Lösungsmittel
DMF : Dimethylformamid

Beispiele 28-32:

2-(N-Piperidinyl)-naphthacen-5,12-dion:

1 g (3,62 mMol) 2-Fluor-naphthacen-5,12-dion, 0,92 g (10,86 mMol) Piperidin, 1,50 g (10,86 mMol) Kaliumcarbonat und 10 ml DMSO werden während 45 Min bei 60 °C Badtemperatur gerührt. Nach dem Abkühlen wird das Gemisch auf Wasser ausgetragen und das orange Produkt abfiltriert. Die Kristalle werden in Tetrahydrofuran (THF)/Toluol gelöst, die Lösung über Natriumsulfat getrocknet und eingedampft. Der Rückstand wird aus Toluol/Pentan umkristallisiert. Ausbeute: 1,11 g (90 %); Smp. 230-233 °C.

Analog wird in den-Beispielen 29-32 verfahren (siehe Tabelle 3.

Tabelle 3

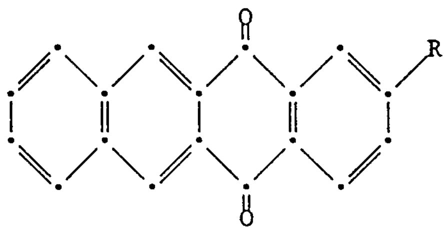

| Beispiel | R | Reaktionszeit (Stunden) | Badtemperatur (°C) | Ausbeute (%) | Smp. (°C) |
|---|---|---|---|---|---|
| 29 | $-N(Et)_2$ [1)] | 25 | 50 | 84 | 170-175 |
| 30 | $-N(Bu)_2$ [2)] | 24 | 60 | 69 | 167-170 |
| 31 | 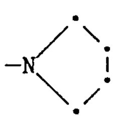 | 1/2 | 60 | 71 | 245-250 |
| 32 | 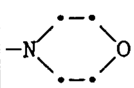 | 7 | 60 | 88 | 230-235 |

1) Ethyl
2) Butyl

Beispiel 33:

2-(Phenylsulfonyl-)-naphthacen-5,12-dion:

1 g (3,62 mMol) 2-Fluor-naphthacen-5,12-dion, 0,89 g (5,43 mMol) Benzolsulfinsäure-Natriumsalz (Natrium-Benzolsulfinat) und 10 ml DMSO werden während 3 Stunden bei 100 °C gerührt. Nach dem Abkühlen wird das Gemisch auf Wasser ausgetragen. Die Kristalle werden abfiltriert und in THF/Toluol gelöst. Die Lösung wird über Natriumsulfat getrocknet und eingedampft. Der Rückstand wird aus THF/Toluol/Pentan umkristallisiert. Ausbeute: 1,35 g (94 %), Smp. >260 °C.

Beispiel 34:

2-(Methylsulfonyl-)-naphthacen-5,12-dion:

Analog Beispiel 33 wird mit Natrium-Methansulfinat durch sechsstündiges Rühren bei 80 °C 2-(Methylsulfonyl-)-naphthacen-5,12-dion hergestellt. Ausbeute: 57 %.

Beispiel 35: Methyl-(2-naphthacen-5,12-dionyl)-sulfoxid

a) 2-Methylthio-naphthacen-5,12-dion:
3,62 mMol 2-Fluor-naphthacen-5,12-dion (hergestellt gemäss US-PS 4,522,754), 3,98 mMol $NaSCH_3$, 10,86 mMol Kaliumcarbonat und 10 ml DMSO werden während 3 Min. bei 25 °C gerührt. Das Gemisch wird auf Wasser ausgetragen. Die Kristalle werden abfiltriert und in Tetrahydrofuran/Toluol gelöst, die Lösung über Natriumsulfat getrocknet und eingedampft. Der Rückstand wird aus THF/Toluol/Pentan umkristallisiert. Ausbeute 1 g (83 %); Smp. 195-196 °C.
b) Methyl-(2-naphthacen-5,12-dionyl)-sulfoxid:
10 g (32,86 mMol) 2-Methylthio-naphthacen-5,12-dion, 3,72 g (32,86 mMol) 30 % $H_2O_2$ und 110 ml Eisessig werden während 4 Std. bei 80 °C gerührt. Das Gemisch wird abgekühlt und in Methylenchlorid/Wasser aufgenommen. Die organische Phase wird abgetrennt, dreimal mit Wasser und dreimal mit $NaHCO_3$-Lösung gewaschen, über Natriumsulfat getrocknet und eingeengt. Durch Chromatographie (Laufmittel: 15 % Aceton, 85 % $CH_2Cl_2$) über Kieselgel werden 7,72 g (73 %) reines Sulfoxid erhalten, Smp. 260-263 °C.

Beispiele 36-40:

(2-Naphthacen-5,12-dionyl-)-malonsäuredimethylester:

50 g (181 mMol) 2-Fluor-naphthacen-5,12-dion, 71,74 g (543 mMol) Malonsäuredimethylester, 75,04 g (543 mMol) Kaliumcarbonat und 500 ml DMSO werden während 2 Std. bei 100 °C Badtemperatur gerührt. Nach dem Abkühlen wird das Gemisch auf verdünnte Salzsäure ausgetragen. Der Niederschlag wird abfiltriert, mit Wasser und Methanol gewaschen, dann aus Toluol umkristallisiert. Ausbeute 63,97 g (91 %); Smp. 211-215 °C.

Analog wird in den Beispielen 37 bis 40 verfahren (siehe Tabelle 4):

Tabelle 4

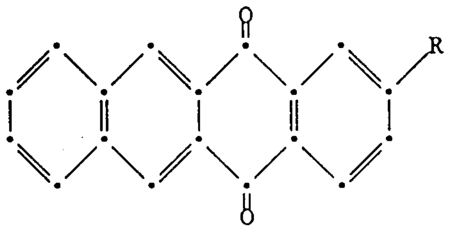

| Beispiel | R | Reaktionszeit (Stunden) | Badtemperatur (°C) | Ausbeute (%) | Smp. (°C) |
|---|---|---|---|---|---|
| 37 | 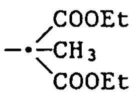 | 2 | 100 | 79 | 134-140 |
| 38 | 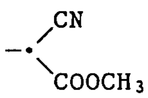 | 24 | 60 | 75 | 210-215 |
| 39 | 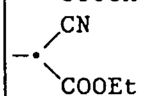 | 3 | 60 | 73 | 212-217 |
| 40 | 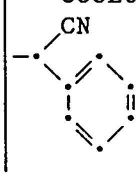 | 42 | 60 | 44 | 220-223 |

Beispiel 41:

(2-Naphthacen-5,12-dionyl-)-essigsäure:

8 g (20,6 mMol) (2-Naphthacen-5,12-dionyl-)-malonsäuredimethylester (Beispiel 36), 60 ml Eisessig und 60 ml konzentrierte HCl-Lösung werden während 4,5 Std. am Rückfluss gerührt. Das Gemisch wird auf Eis/Wasser ausgetragen. Die Kristalle werden abfiltriert, mit Diethylether gewaschen und aus THF/Pentan umkristallisiert. Ausbeute 4,88 g (75 %); Smp. >220 °C.

Beispiel 42

1-Nitro-naphthacen-5,12-dion:

30 g (147,7 mMol) 5-Nitronaphtho-1,4-chinon, 58,02 g (ca. 220 mMol) 1,2-Dibrombenzocyclobuten (verunreinigt mit etwas 1-Jod-2-brombenzocyclobuten) und 300 ml Toluol werden während 2 Tagen am Rückfluss gerührt, wobei die entstehende Bromwasserstoffsäure zusammen mit etwas Toluol abdestilliert wird und das dabei verlorene Toluol durch frisches ersetzt wird. Das Gemisch wird auf 25 °C abgekühlt, der Niederschlag abfiltriert, mit Toluol gewaschen und im Vakuum bei 140 °C getrocknet. Ausbeute: 43,46 g (97 %); Smp. >250 °C. Das Produkt kann aus γ-Butyrolacton umkristallisiert werden; Ausbeute: 77 %.

Beispiele 43-51:

1-Phenoxy-naphthacen-5,12-dion:

3 g (9,9 mMol) 1-Nitronaphthacen-5,12-dion (Beispiel 42), 2,73 g (19,8 mMol) Kaliumcarbonat, 1,39 g (14,8 mMol) Phenol und 30 ml DMSO werden während 6 Std. bei 100 °C Badtemperatur gerührt. Nach dem Abkühlen wird das Gemisch zwischen Methylenchlorid/verdünnter Salzsäure verteilt. Die organische Phase wird abgetrennt, mit Wasser gewaschen, über Natriumsulfat getrocknet, und eingedampft. Durch Chromatographie an Kieselgel (Laufmittel: Methylenchlorid) wird das Produkt rein erhalten; Ausbeute 2,52 g (73 %); Smp 195-200 °C.

Analog wird in den Beispielen 44-51 verfahren (siehe Tabelle 5)

Tabelle 5

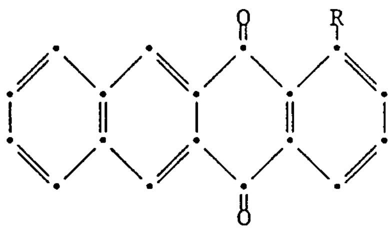

| Beispiel | R | Reaktionszeit (Stunden) | Badtemperatur (°C) | Ausbeute (%) | Smp. (°C) |
|---|---|---|---|---|---|
| 44 | $-OCH_3$ | 23 | 100 | 68 | 260-262 |
| 45 | $-SCH_3$ *) | $^1/_3$ | 25 | 56 | 260-265 |
| 46 | $-S-CH_2CH_3$ | 1 | 25 | 48 | 200-205 |
| 47 | $-S-CH_2-CH_2-OH$ | 1,5 | 25 | 30 | 250-255 |
| 48 | 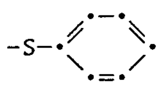 | 1,5 | 25 | 54 | 255-258 |
| 49 | 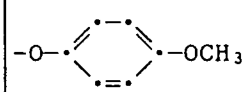 | 24 | 80 | 39 | 210-215 |
| 50 | 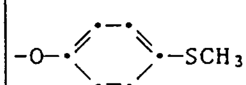 | 20 | 80 | 65 | 205-206 |
| 51 | 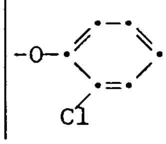 | 20 | 80 | 28 | 205-206 |

*) Hier wird anstelle des Mercaptans $NaSCH_3$ verwendet.

Beispiel 52:

2-Fluor-3-methoxy-naphthacen-5,12-dion:

1 g (3,4 mMol) 2,3-Difluornaphthacen-5,12-dion, 0,94 g (6,8 mMol) Kaliumcarbonat, 3,24 g Methanol und 20 ml Tetrahydrofuran (THF) werden 18 Std. bei 25 °C und 3 Tage am Rückfluss gerührt. Das Gemisch wird abgekühlt und mit verdünnter Salzsäure versetzt. Das Produkt wird abfiltriert, mit Wasser gewaschen, getrocknet und mit Methylenchlorid an Kieselgel chromatographiert. Nach der Sublimation bei 1,3x $10^{-1}$ bar/180-310 °C werden 0,26 g (25 %) reines Produkt erhalten, Smp. >250 °C.

Beispiel 53:

2,3-Dimethoxy-naphthacen-5,12-dion:

1 g (3,4 mMol) 2,3-Difluor-naphthacen-5,12-dion, 0,94 g (6,8 mMol) $K_2CO_3$, 2,18 g Methanol und 20 ml DMSO werden während 22 Std. bei 110 °C gerührt. Das Gemisch wird auf verdünnte Salzsäure ausgetragen. Das Produkt wird abfiltriert, viermal mit Wasser gewaschen, getrocknet und aus $CH_2Cl_2$/Pentan umkristallisiert; Ausbeute 0,72 g (67 %); Smp. >250 °C.

Beispiel 54:

2-Fluor-3-(N-morpholino-)-naphthacen-5,12-dion:

1 g (3,4 mMol) 2,3-Difluor-naphthacen-5,12-dion, 0,94 g (6,8 mMol) Kaliumcarbonat, 1,48 g (17 mMol) Morpholin und 20 ml THF werden während 23 Std. am Rückfluss gerührt. Das Gemisch wird auf Wasser ausgetragen. Das Produkt wird abfiltriert, mit Wasser gewaschen, getrocknet und aus Dioxan umkristallisiert; Ausbeute 0,78 g (64 %); Smp. >240 °C.

Beispiel 55:

2-(3-Fluor-naphthacen-5,12-dionyl)-malonsäuredimethylester:

1 g (3,4 mMol) 2,3-Difluor-naphthacen-5,12-dion, 0,94 g (6,8 mMol) Kaliumcarbonat, 0,89 g (6,8 mMol) Malonsäuredimethylester und 20 ml DMSO werden während 22 Stunden bei 50 °C gerührt. Das Gemisch wird auf verdünnte Salzsäure ausgetragen und mit THF/Toluol extrahiert. Die organische Phase wird mit Wasser gewaschen, über Natriumsulfat getrocknet, und eingedampft. Durch Chromatographie über Kieselgel (Laufmittel: 1 % Aceton/99 % $CH_2Cl_2$) wird das Produkt gereinigt; Ausbeute 1,25 g (91 %); Smp. 225-230 °C.

Beispiel 56:

2-Cyano-naphthacen-5,12-dion:

10 g (33,2 mMol) Naphthacen-5,12-dion-2-carbonsäureamid, 10,18 g (66,4 mMol) $POCl_3$ und 200 ml DMF werden bei 10 °C, dann bei 25 °C während 2 Stunden gerührt. Das Gemisch wird auf Eiswasser ausgetragen. Der Niederschlag wird abfiltriert, dreimal mit Wasser gewaschen und getrocknet. Aus o-Dichlorbenzol umkristallisiert, werden 7,35 g (78 %) Rohprodukte erhalten.

IR-Spektrum (KBr): 1678 $cm^{-1}$: Chinon; 2240 $cm^{-1}$: CN

Massenspektrum: M/e = 283 ($M^+$) (Basispeak); 255; 227; 226; 100.

Beispiel 57:

2-(Trifluormethyl-)-naphthacen-5,12-dion:

5,65 g (25 mMol) 6-(Trifluormethyl-)-1,4-naphthochinon, 9,82 g (ca 37 mMol) 1,2-Dibrombenzocyclobuten (verunreinigt mit etwas 2-Brom-1-jodbenzocyclobuten) und 100 ml Xylol werden während 16 Stunden mit Wasserabscheider am Rückfluss gehalten. Das Gemisch wird abgekühlt und der Niederschlag abfiltriert umd mit Xylol gewaschen. Ausbeute 5,82 g (71 %); Smp. 253-254 °C. In den Beispielen 58 bis 61 wird

analog verfahren.

Beispiel 58: 2,3-bis-(Trifluormethyl-)-naphthacen-5,12-dion; Ausbeute 59 %; Smp. >280 ° C.

Beispiel 59: 1-(Trifluormethyl-)-naphthacen-5,12-dion-3-carbonsäuremethylester; Ausbeute 60 %; Smp. 234-235 ° C.

Beispiel 60: 2-Ethoxy-naphthacen-5,12-dion-3-carbonsäuremethylester; Ausbeute 30 %; Smp. 192-194 ° C.

Beispiel 61: 2-Ethoxy-naphthacen-5,12-dion-3-dicarbonsäurediethylester; Ausbeute 41 %; Smp. 163-164 ° C.

Ausgangsprodukte:

Die substituierten Naphthochinone der Beispiele 57 bis 60 werden durch Diels-Alder-Reaktion entsprechender α-Pyrone mit p-Benzochinon erhalten, wobei die Trifluormethylderivate durch Fluorierung mit $HF/SF_4$ der Carbonsäureethylester hergestellt werden. Das Naphthochinon von Beispiel 60 wird durch Diels-Alder-Reaktion von 1-Ethoxy-2,4-dicarbethoxy-1,4-butadien mit p-Benzochinon erhalten.

Beispiele 62-64:

Eine Mischung aus 1 Mol entsprechend substituiertem Dibrom- bzw. Bromjod-benzocyclobuten*, 1,5 Mol Naphthochinon und 7 Liter Lösungsmittel wird während 4-16 Stunden am Rückfluss erhitzt. Nach dem Abkühlen wird das ausgefallene Naphthacenchinon abfiltriert und durch Kristallisation bzw. Sublimation im Hochvakuum gereinigt (siehe Tabelle 6)

Tabelle 6

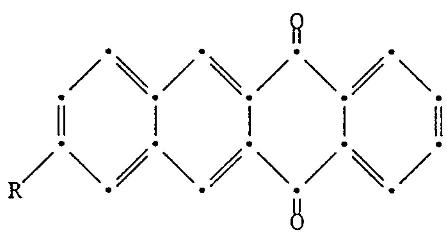

| Beispiel No. | R | Lösungsmittel | Reaktionszeit (Stunden) | Ausbeute (%) | Smp. (°C) | Massenspektrum ($M^+$/ %) |
|---|---|---|---|---|---|---|
| 62[1] | -Br | Xylol | 16 | 50 | >270 | 336/90 |
| 63[1] | $-COOCH_3$ | Dichlorbenzol | 16 | 10 | 268 | 316/100 |
| 64[2] | $-NO_2$ | Dichlorbenzol | 4 | 22 | >270 | 303/100 |

[1]) Aus Toluol umkristallisiert
[2]) Bei 200°C sublimiert
*) Hergestellt durch Umsetzung entsprechend substituierter o-Dihalogenmethyl-benzole mit NaI in Acetonitril bei 80°C.

Beispiele 65-95:

Eine Mischung aus 0,1 Mol entsprechend substituiertem Bis-dibromomethylbenzol, 0,12-0,2 Mol gegebenenfalls substituiertem Naphthochinon, 0,6 Mol Natriumjodid und 1000 ml Aceton bzw. Acetonitril wird unter Rühren und $N_2$-Atmosphäre während 2-6 Stunden am Rückfluss gekocht. Nach dem Abkühlen wird

der ausgefallene Niederschlag abfiltriert und mit Wasser digeriert. Das auf diese Weise erhaltene substituierte Naphthacendion wird zur Weiterreinigung entweder umkristallisiert oder im Hochvakuum sublimiert (vgl. Tabellen 7, 8 und 9).

Tabelle 7

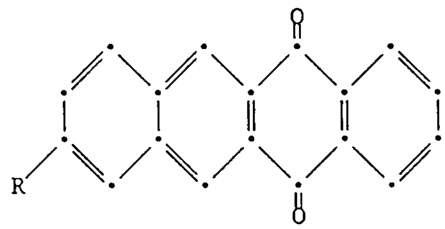

| Beispiel No. | R | Lösungsmittel | Reaktionszeit (Stunden) | Umkristallisation/ Sublimation | Ausbeute (%) | Smp. (°C) | Massenspektrum ($M^+$/ %) |
|---|---|---|---|---|---|---|---|
| 65[a)] | $Me_3Si$ | Acetonitril | 5 | Sublim. 150°C | 79 | 168-170 | |
| 66 | F | Acetonitril | 2 | Sublim. 200°C | 68 | >270 | 276/100 |

a) Filtrat wird zur Trockne verdampft, Rückstand in $CH_2Cl_2$ gelöst und mit $NaHSO_3$ gewaschen und getrocknet. Anschliessend wird der Rückstand einer Wasserdampfdestillation unterworfen und die nichtflüchtigen Teile mit Ether extrahiert, getrocknet und dann sublimiert.

Tabelle 8

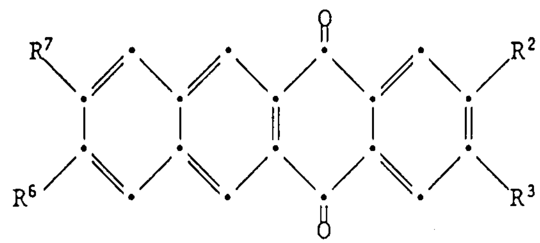

| Beispiel No. | $R^6$ | $R^7$ | $R^2$ | $R^3$ | Lösungsmittel | Reaktionszeit (Stunden) | Umkristallisation/ Sublimation | Ausbeute (%) | Smp. (°C) | Massenspektrum ($M^+$/ %) |
|---|---|---|---|---|---|---|---|---|---|---|
| 67 | $OCH_3$ | H | $CF_3$ | $CF_3$ | Acetonitril | 5 | Sublim. 200°C | 33 | >280 | 424/100 |
| 68 | $OCH_3$ | H | COOEt | COOEt | Acetonitril | 5 | Sublim. 180°C | 40 | 214-216 | 432/90 |
| 69 | $OCH_3$ | H | $CH_3$ | $CH_3$ | Aceton | 5 | Sublim. 180°C | 13 | 268-270 | 316/100 |
| 70 | $Me_3Si$ | $Me_3Si$ | H | H | Acetonitril | 3 | Sublim. 180°C | 70 | 196-197 | 405/55 |
| 71 b) | $Me_3Si$ | $Me_3Si$ | COOEt | COOEt | Acetonitril | 3 | Isopropylether | 64 | 138-139 | 546/80 |
| 72 | CN | CN | COOEt | COOEt | $CH_3CN$ | 5 | Sublim. | 44 | >270 | 452/10 |
| 73 | CN | CN | H | H | $CH_3CN$ | 5 | Sublim. | 71 | >270 | 308/100 |
| 74 | Br | Br | H | H | $CH_3CN$ | 4 | Sublim. | 60 | >250 | 416/100 |
| 75 | Br | Br | COOEt | COOEt | $CH_3CN$ | 4 | Sublim. | 53 | >250 | 560/64 |
| 76 | Br | Br | $CF_3$ | $CF_3$ | $CH_3GN$ | 4 | Sublim. | 58 | >250 | 552/100 |

Tabelle 8 (Fortsetzung)

| Beispiel No. | $R^6$ | $R^7$ | $R^2$ | $R^3$ | Lösungsmittel | Reaktionszeit (Stunden) | Umkristallisation/ Sublimation | Ausbeute (%) | Smp. (°C) | Massenspektrum ($M^+$/ %) |
|---|---|---|---|---|---|---|---|---|---|---|
| 77 | $CF_3$ | $CF_3$ | Cl | COOEt | $CH_3CN$ | 6 | Sublim. | 68 | >270 | 500/60 |
| 78 | $CF_3$ | $CF_3$ | $CF_3$ | $CF_3$ | $CH_3CN$ | 8 | Sublim. | 70 | >280 | 530/100 |
| 79 | $CF_3$ | H | COOEt | COOEt | Aceton | 5 | Sublim. | 60 | 216-217 | 470/40 |
| 80 | $CF_3$ | H | $CF_3$ | $CF_3$ | Aceton | 5 | Sublim. | 55 | 261-262 | 462/100 |
| 81 | Cl | Cl | H | H | $CH_3CN$ | 5 | Sublim. | 54 | >250 | 326/100 |
| 82 | Cl | Cl | $CF_3$ | $CF_3$ | $CH_3CN$ | 5 | Sublim. | 41 | >250 | 462/100 |
| 83 | Cl | Cl | COOEt | COOEt | $CH_3CN$ | 5 | Sublim. | 68 | >250 | 470/48 |
| 84 | COOMe | COOMe | H | H | $CH_3CN$ | 4 | Sublim. | 67 | >250 | 374/48 |
| 85 | COOMe | COOMe | COOEt | COOEt | $CH_3CN$ | 4 | Sublim. | 70 | 238-239 | 518/88 |
| 86 | COOMe | COOMe | $CF_3$ | $CF_3$ | $CH_3CN$ | 5 | Sublim. | 72 | >250 | 510/40 |
| 87 | $Me_3Si$ | $Me_3Si$ | $CF_3$ | $CF_3$ | $CH_3CN$ | 5 | Isopropylether | 30 | 143-145 | 538/60 |

b) Filtrat wird zur Trockne verdampft, Rückstand in Ether gelöst und mit $NaHSO_3$ gewaschen, getrocknet. Etherrückstand wird mit $CH_2Cl_2$ über eine Kieselgelsäule filtriert.

Tabelle 9

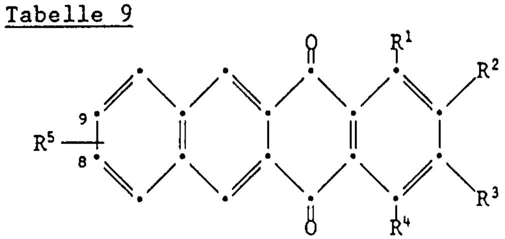

| Beispiel No. | $R^1$ | $R^2$ | $R^3$ | $R^4$ | $R^5$ | Lösungsmittel | Reaktionszeit (Stunden) | Sublimation (°C) | Ausbeute (%) | Smp. (°C) | Massenspektrum ($M^+$/ %) |
|---|---|---|---|---|---|---|---|---|---|---|---|
| 88 | H | $COOCH_3$ | H | H | $OCH_3$ | Aceton | 5 | 180 | 50 | 246-248 | 346/95 |
| 89 | $NO_2$ | H | H | H | $OCH_3$ | Aceton | 5 | 180 | 28 | >280 | 333/95 |
| 90 | $CF_3$ | H | $COOCH_3$ | H | $OCH_3$ | Aceton | 5 | 200 | 80 | 235-240 | 414/100 |
| 91 | COOEt | OEt | COOEt | H | $OCH_3$ | Aceton | 5 | 220 | 16 | 230-232 | 476/60 |
| 92 | H | $COOCH_3$ | H | $CF_3$ | $CF_3$ | $CH_3CN$ | 6 | 240 | 50 | >280 | 452/100 |
| 93 | H | Cl | COOEt | H | $CF_3$ | $CH_3CN$ | 6 | 230 | 60 | >280 | 432/70 |
| 94 1) | H | F | H | H | F | Fluorbenzol | 4 | 225 | 47 | 278-280 | 294/100 |
| 95 | H | Cl | H | H | F | $CH_3CN$ | 6 | 270 | 26 | >280 | 310/100 |

Diese Anthracendione liegen etwa als 1:1 Gemische der beiden möglichen Isomeren vor ($-OCH_3$ in 8- bzw. 9-Stellung gebunden).

1)
Katalysator: $AlCl_3$.

B) Anwendungsbeispiele

Beispiel 96: Photohärtung eines Acrylat-Gemisches zur Herstellung eines Reliefbildes.

Es wird eine photohärtbare Zusammensetzung hergestellt durch Mischen der folgenden Komponenten:

| | Feststoffgehalt |
|---|---|
| 150,30 g Scripset 540[1)] (30 %-ige Lösung in Aceton)<br>48,30 g Trimethylolpropantriacrylat<br>6,60 g Polyethylenglykoldiacrylat<br>0,08 g Kristallviolett | 45,1 g<br>48,3 g<br>6,6 g |
| 205,28 g | 100,0 g |

1) Polystyrol-Maleinsäurehalbester-Copolymer (Monsanto)

Portionen dieser Zusammensetzung werden mit 0,2 % (bezogen auf die Zusammensetzung) der in der folgenden Tabelle angegebenen Photoinitiatoren vermischt. Alle Operationen werden unter Rotlicht oder Gelblicht ausgeführt.

Die mit Initiator versetzten Proben werden mit einem Spiralrakel von 150 µm auf 200 µm Aluminiumfolie (10 x 15 cm) aufgetragen. Das Lösungsmittel wird durch Erwärmung auf 60 °C während 15 Minuten im Umluftofen entfernt. Es resultiert eine Trockenschichtdicke von etwa 35 µm. Auf die Schicht wird eine 76 µm dicke Polyesterfolie gelegt und auf diese ein standardisiertes Testnegativ mit 21 Stufen verschiedener optischer Dichte (Stouffer-Keil) gelegt. Darüber wird eine zweite Polyesterfolie gelegt und das so erhaltene Laminat mittels Vakuum auf einer Metallplatte fixiert. Die Probe wird dann mit einer 5 KW-Metallhalogenid-Lampe (Typ MO 23) im Abstand von 30 cm belichtet und zwar in einer ersten Testreihe 20 Sekunden und einer zweiten Testreihe 40 Sekunden. Nach der Belichtung werden die Folien und die Maske entfernt, die belichtete Schicht in einem Ultraschallbad 120 Sekunden mit Entwickler A 2 Minuten lang entwickelt und anschliessend bei 60 °C 15 Minuten im Umluftofen getrocknet. Die Empfindlichkeit des verwendeten Initiatorsystems wird durch die Angabe der letzten klebefrei abgebildeten Keilstufe charakterisiert. Je höher die Zahl der Stufen ist, desto empfindlicher ist das System. Eine Erhöhung um zwei Stufen bedeutet dabei etwa eine Verdopplung der Härtungsgeschwindigkeit. Die Ergebnisse sind in Tabelle 10 angegeben. Entwickler A enthält 15 g Natriummetasilikat•9 $H_2O$; 0,16 g KOH; 3 g Polyethylenglykol 6000; 0,5 g Lävulinsäure und 1000 g deionisiertes Wasser.

Tabelle 10

| Photoinitiator | Zahl der abgebildeten Stufen | |
|---|---|---|
| Naphthacendion von Beispiel | nach 20 s | nach 40 s Belichtung |
| 2 | 3 | 5 |
| 7 | 2 | 4 |
| 35 | 3 | 5 |
| 37 | 2 | 4 |
| 44 | 5 | 7 |
| 88 | 1 | 2 |
| A[1] | 3 | 5 |
| B[2] | 2 | 4 |

1) 2-Anthracen-5,12-dioncarbonsäuremethylester

2) 6-Methoxy-anthracen-5,12-dion

Beispiel 97:

Eine Lösung der Zusammensetzung:

| | |
|---|---|
| Ethylcellosolve | 71,00 g |
| Scripset 550*) | 14,00 g |
| Trimethylolpropantriacrylat | 15,00 g |
| Polyethylenglykol-200-diacrylat | 2,00 g |

*) Styrol-Maleinsäuremonoester-Copolymer ($M_w$ = 10'000, Säurezahl 190), Hersteller Monsanto

wird in gleiche Portionen von jeweils 10 Gramm aufgeteilt. In dieser Lösung werden jeweils 0,05 g Naphthacendion sowie 0,5 g Glycerin unter Rotlicht gelöst.

Mit einem Drahtrakel werden Beschichtungen in 12 Mikrometer Nassfilmdicke auf transparenter Polyesterfolie aufgebracht. Die nassen Filme werden in einem Umluftofen bei 80 °C für 30 Minuten getrocknet.

Durch Eintauchen in eine Lösung aus

| | |
|---|---|
| Mowiol 4-88 (Polyvinylalkohol) | 30,00 g |
| Brij 35 (10 % in Wasser)**) | 15,00 g |
| deionisiertes Wasser | 250,00 g |

**) Polyoxyethylen-laurylether (Netzmittel, Hersteller Atlas Powder)

und anschliessendes Trocknen bei 80 °C im Umluftofen wird eine Sauerstoffsperrschicht in 0,5 Mikrometer Dicke aufgebracht.

Der trockene Film wird mit einer 5000 W Quecksilberlampe (MO 33 Fa. Staub, Neu Isenburg) durch einen Stufenkeil mit Inkrementen von 0,15 (log O.D.) belichtet und anschliessend in einer Lösung der Zusammensetzung

| | |
|---|---|
| Natrium-metasilikat-nonahydrat | 15,00 g |
| Strontiumhydroxid-octahydrat | 0,30 g |
| Polyglykol 6000 | 3,00 g |
| Lävulinsäure | 0,50 g |
| deionisiertes Wasser | 1000,00 g |

zu einem Reliefbild entwickelt. Die Lichtintensität wird mit einem Powermeter der Fa. Oriel mit 365 nm Sensor gemessen. Tabelle 11 zeigt die für die zur Erreichung der Stufe 7 des Stufenkeils benötigte Belichtungsenergie.

Tabelle 11

| Naphthacendion von Beispiel | UV-Spektrum (Extinktion bei $\lambda_{max}$) | $WP_7$ ($mJ/cm^{-2}$) |
|---|---|---|
| 37 | $\epsilon$ = 6100, $\lambda_{max}$: 398 nm Schulter bei 414 nm | 660 |

Beispiel 98: Photometallierung

Ein Copolymer aus 65 Mol% Hydroxypropylmethacrylat und 35 Mol% Methylmethacrylat mit einem Molekulargewicht von 120'000 Dalton (gemessen durch Lichtstreuung in Dioxan bei 25 °C) wird in DMF gelöst und mit 5 Mol% Anthracendion gemäss Beispiel 37 (bezogen auf Polymerhydroxylgruppen) versetzt. Durch Aufrakeln der Lösung auf einen Polyesterträger und anschliessende Trocknung bei 80 °C für 2 Stunden in einem Umluftofen werden Filme hergestellt. Die Filme werden auf einem thermostatisierbaren Vakuumheiztisch bei 50 °C unter einem Negativ mit einer HG-Hochdrucklampe mit 40 $mW/cm^{-2}$ Intensität

belichtet. Man erhält ein dunkles, negatives Abbild der Vorlage, das in einem Abscheidungsbad der Zusammensetzung

| | |
|---|---|
| $CuSO_4$ x $5H_2O$ | 0,0665 Mol/l |
| HCOH | 0,0467 Mol/l |
| Quadrol | 0,0599 Mol/l |
| NaOH | pH 12,6 |
| NaCN | 25 mg/l |
| 2-Mercaptobenzthiazol | 10 mg/l |

bei 45 °C zu einem Kupferbild verstärkt wird.

Beispiel 99: Sensibilisierung der 2+2 Cycloaddition

In einer 20 % (w/w) Lösung eines Copolymers mit Molekulargewicht 140'000 Dalton (gemessen durch Lichtstreuung in Dioxan bei 25 °C) aus 20 Mol% Ethylacrylat und 80 Mol% N-(5-methyl-3-oxa-4-oxohexen-5-yl)dimethylmaleinimid (hergestellt nach dem Verfahren in Angew. Makromol. Chem. 115 (1983) 163ff) werden 5 % (w/w) 2-Methoxy-anthracen-5,12-dion gelöst. Die Lösung wird als Film mit einem Drahtrakel in 24 µm Nassfilmdicke auf ein kupferbeschichtetes Glasfaser-Epoxidlaminat aufgerakelt und bei 80 °C 1 Stunde lang getrocknet. Der trockene Film wird mit einer 5000 W Quecksilberlampe (MO 33, Fa. Staub) durch einen Stufenkeil mit Inkrementen von 0.15 (log O.D.) belichtet und anschliessend in 1,1,1-Trichlorethan zu einem Reliefbild entwickelt. Die zur Erreichung der Stufe 7 des Stufenkeils benötigte Belichtungsenergie beträgt 231 mJ x $cm^{-2}$.

## Patentansprüche

**1.** Verbindungen der Formel I

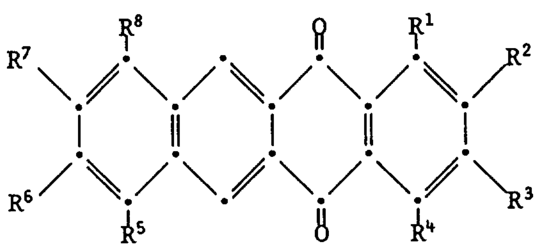

(I),

worin $R^5$ und $R^8$ H sind und

a) $R^1$, $R^2$, $R^3$, $R^4$ H sind,

und $R^6$ und $R^7$ je für H stehen und mindestens einer der Reste $R^6$ und $R^7$ unabhängig voneinander einen Rest aus der Gruppe unsubstituiertes oder mit Halogen, -CN, Furfuryl, $-NR^9R^{10}$, $-OR^9$, $-SR^9$ oder $-COOR^9$ substituiertes $C_1$-$C_{20}$-Alkyl$\text{-}(X)_p$ mit Ausnahme von Methoxy, $C_2$-$C_{18}$-Alkenyl$\text{-}(X)_p$, $C_2$-$C_{18}$-Alkinyl$\text{-}(X)_p$, $C_3$-$C_8$-Cycloalkyl$\text{-}(X)_p$, ($C_1$-$C_{12}$-Alkyl)-$C_3$-$C_8$-cycloalkyl$\text{-}(X)_p$, $C_3$-$C_8$-Cycloalkyl-$C_rH_{2r}\text{-}(X)_p$, ($C_1$-$C_{12}$-Alkyl)-$C_3$-$C_8$-cyclo alkyl-$C_rH_{2r}\text{-}(X)_p$, Phenyl$\text{-}(X)_p$, ($C_1$-$C_{12}$-Alkyl)phenyl$\text{-}(X)_p$, Phenyl-$C_rH_{2r}\text{-}(X)_p$,($C_1$-$C_{12}$-Alkyl)phenyl-$C_rH_{2r}\text{-}(X)_p$ darstellen,

r für 1 oder 2 und p für 1 stehen und X -O-, -SO- oder $-SO_2$-bedeutet, oder $R^6$, $R^7$ unabhängig einen Rest aus der Gruppe Halogen, $-NO_2$, $-CF_3$, -CN, $-NR^9R^{10}$, $-COOR^9$, $-CONR^9R^{10}$, -COCl, -SH, $-Si(C_1\text{-}C_4\text{-Alkyl})_3$ oder $-O\text{-}(C_mH_{2m}\text{-}O)_nR^{11}$ bedeuten,

oder $R^6$ und $R^7$ zusammen für -CO-O-CO-oder $-CO-NR^9-CO-$ stehen,

$R^9$ und $R^{10}$ unabhängig voneinander H, $C_1$-$C_{12}$-Alkyl, Phenyl oder $\text{-}(C_mH_{2m}\text{-}O)_qR^{11}$, oder $R^9$ und $R^{10}$ zusammen Tetramethylen, Pentamethylen, 3-Oxapentylen oder $-CH_2CH_2NR^9CH_2CH_2-$ bedeuten, $R^{11}$ H oder $C_1$-$C_{12}$-Alkyl ist,

m für eine Zahl von 2 bis 4, n für eine Zahl von 2 bis 20 und q für eine Zahl von 1 bis 20 stehen, oder

b) $R^1$ bis $R^4$, $R^6$ und $R^7$ unabhängig voneinander H oder einen der zuvor definierten Reste einschliesslich Methoxy darstellen, wobei $R^1$ und $R^4$ auch Reste mit X in der Bedeutung von -S- sind

und mindestens eines von $R^1$ bis $R^4$ sowie $R^6$ und $R^7$ einen Rest bedeuten, mit Ausnahme von $R^2$ und $R^6$ oder $R^7$ gleich -F, oder

c) $R^6$ und $R^7$ für H stehen, mindestens eines von $R^1$, $R^2$, $R^3$ und $R^4$ einen Rest bedeuten,

und $R^1$ und $R^4$ unabhängig voneinander H oder einen Rest aus der Gruppe $-NO_2$, $-CF_3$, -CN, $-COOR^9$, $-CONR^9R^{10}$, -COCl, $-Si(C_1\text{-}C_4\text{-Alkyl})_3$ $-S{(C_mH_{2m}\text{-}O)}_nR^{11}$, $-O{(C_mH_{2m}\text{-}O)}_nR^{11}$, unsubstituiertes oder wie zuvor unter a) definiert substituiertes $C_1$-$C_{20}$-Alkyl-$X^1$-; $C_2$-$C_{18}$-Alkenyl$(X^1)_p$,$C_2$-$C_{18}$-Alkinyl$(X^1$-$_p$, $C_3$-$C_8$-Cycloalkyl$(X^1)_p$,

($C_1$-$C_{12}$-Alkyl)-$C_3$-$C_8$-cycloalkyl$(X^1)_p$, $C_3$-$C_8$-Cycloalkyl-$C_rH_{2r}(X^1)_p$,($C_1$-$C_{12}$-Alkyl)-$C_3$-$C_8$-cycloalkyl-$C_rH_{2r}(X^1)_p$; Phenyl$(X^1)_p$

oder ($C_1$-$C_{12}$-Alkyl)phenyl$(X^1)_p$, Phenyl-$C_rH_{2r}(X)_p$, $C_1$-$C_{12}$-Alkyl)phenyl-$C_rH_{2r}(X)_p$; wie zuvor unter a) definiert substituiertes $C_1$-$C_{20}$-Alkylthio, Phenyloxy, ($C_1$-$C_{12}$-Alkyl)phenyloxy oder mit Halogen, -CN, $-NR^9R^{10}$, $-SR^9$, $-OR^9$ oder $COOR^9$ substituiertes $C_1$-$C_{20}$-Alkoxy darstellen; wobei $R^1$ nicht -COOH ist, wenn $R^2$, $R^3$ und $R^4$ H bedeuten,

und $R^2$ und $R^3$ unabhängig voneinander H oder einen Rest aus der Gruppe unsubstituiertes oder wie zuvor unter a) definiert substituiertes $C_5$-$C_{20}$-Alkyl, $C_3$-$C_{20}$-Alkoxy, $C_1$-$C_{20}$-Alkyl-$X^1$-; Phenyl-X-, ($C_1$-$C_{12}$-Alkyl)phenyl$(X)_p$, ($C_1$-$C_{12}$-Alkyl)phenyl-$C_rH_{2r}(X)_p$, Phenyl-$C_rH_{2r}(X)_p$, $C_2$-$C_{20}$-Alkenyl$(X)_p$, $C_2$-$C_{20}$-Alkinyl$(X)_p$, $C_3$-$C_8$-Cycloalkyl$(X)_p$,($C_1$-$C_{12}$-Alkyl)-$C_3$-$C_8$-cycloalky$(X)_p$, $C_3$-$C_8$-Cycloalkyl-$C_rH_{2r}(X)_p$ oder ($C_1$-$C_{12}$-Alkyl)-$C_3$-$C_8$-cycloalkyl-$C_rH_{2r}(X)_p$,

oder eines von $R^2$ und $R^3$ oder $R^2$ und $R^3$ einen Rest aus der Gruppe mit Halogen, -CN, $-NR^9R^{10}$, $OR^9$, $-SR^9$, $-COOR^9$ substituiertes $C_1$-$C_4$-Alkyl oder $C_1$-$C_2$-Alkoxy mit Ausnahme von 2-Hydroxyethyl oder eines von $R^2$ und $R^3$ oder $R^2$ und $R^3$ einen Rest aus der Gruppe SH, $-NO_2$, $-CF_3$, -CN, $-NR^9R^{10}$, $-Si(C_1\text{-}C_4\text{-Alkyl})_3$ oder $-O{(C_mH_{2m}\text{-}O)}_nR^{11}$ darstellen;

oder eines von $R^2$ und $R^3$ Halogen, $-COOR^9$, $-CONR^9R^{10}$ oder -COCl und das andere von $R^2$ und $R^3$ einen wie unter a) definierten Rest darstellen, oder das andere von $R^2$ und $R^3$ H ist, wenn mindestens eines von $R^1$ und $R^4$ einen der zuvor definierten Reste darstellt;

oder $R^2$ und $R^3$ zusammen -CO-O-CO- oder -CO-$NR^9$-CO- und mindestens eines von $R^1$ und $R^4$ einen der zuvor definierten Reste darstellen, oder $R^2$ und $R^3$ zusammen unabhängig voneinander $C_1$-$C_2$-Alkoxy sind, wobei $R^9$, $R^{10}$, $R^{11}$, X, m, n, p, q, r und s, die unter a) angegebenen Bedeutungen haben und $X^1$ -SO- oder $-SO_2$- darstellt, oder

d) $R^6$ und $R^7$ H bedeuten und $R^1$ unsubstituiertes $C_1$-$C_{20}$-Alkylthio, $C_1$-$C_{20}$-Alkoxy, Phenyloxy, Phenylthio, $C_1$-$C_{12}$-Alkylphenyloxy, $C_1$-$C_{12}$-Alkylphenylthio, oder wie unter a) definiert substituiertes Phenylthio oder $C_1$-$C_{12}$-Alkylphenylthio darstellen.

2. Verbindungen gemäss Anspruch 1, worin $R^4$, oder $R^1$ und $R^4$ für H stehen.

3. Verbindungen gemäss Anspruch 1, worin $R^2$ oder $R^3$, oder $R^2$ und $R^3$, einen Rest darstellen.

4. Verbindungen gemäss Anspruch 1, worin $R^1$, $R^2$ und $R^3$ oder $R^1$ und $R^2$ oder $R^1$ und $R^3$ einen Rest darstellen und $R^4$ H bedeutet.

5. Verbindungen gemäss den Ansprüchen 3 oder 4, worin $R^6$ oder $R^7$, oder $R^6$ und $R^7$ H oder einen Rest bedeuten.

6. Verbindungen gemäss Anspruch 1, worin $R^1$ bis $R^4$ H bedeuten;
mindestens einer der Reste $R^6$ oder $R^7$ einen Rest aus der Gruppe unsubstituiertes oder mit F, Cl, Br, -CN, $-NR^9R^{10}$, $-OR^9$, $-SR^9$ oder $-COOR^9$ substituiertes $C_1$-$C_{18}$ Alkylk$(X)_p$, Phenyl$(X)_p$, ($C_1$-$C_6$-Alkyl)-phenyl$(X)_p$, Benzyl$(X)_p$, ($C_1$-$C_6$-Alkyl)benzyl$(X)_p$;
unsubstituiertes $C_3$-$C_{12}$-Alkenoxy oder $C_3$-$C_6$-Alkinoxy, deren Alken- bzw. Alkingruppe nicht an das O-Atom gebunden ist; oder -F, -Cl, -Br, $-NO_2$, $-CF_3$, -CN, $-NR^9R^{10}$, $COOR^9$, $-CONR^9R^{10}$, $-Si(CH_3)_3$ oder $-O{(C_2H_4\text{-}O)}_nR^{11}$ darstellt; $R^{11}$ H oder $C_1$-$C_4$-Alkyl ist;
X -O-, -SO- oder $-SO_2$-, p 1 und n 2 bis 20 darstellen, und wobei $R^9$ und $R^{10}$ je für H, $C_1$-$C_6$-Alkyl, oder $R^9$ und $R^{10}$ zusammen Tetra-oder Pentamethylen, 3-Oxapentylen oder $-CH_2CH_2N(C_1\text{-}C_6\text{-Alkyl})$-$CH_2CH_2$-stehen.

7. Verbindungen gemäss Anspruch 6, worin $R^6$ oder $R^7$ oder $R^6$ und $R^7$ -F, -Cl, -Br, -CN, $-NO_2$, -COOH, $-COO(C_1\text{-}C_4)$-Alkyl, $-CF_3$, $-Si(CH_3)_3$ oder unsubstituiertes oder substituiertes $C_1$-$C_{12}$-Alkyl-X- bedeuten.

**8.** Verbindungen gemäss Anspruch 1, worin $R^4$ H ist;
mindestens einer der Reste $R^1$, $R^2$ und $R^3$ sowie mindestens einer der Reste $R^6$ und $R^7$ einen Rest aus der Gruppe unsubstituiertes oder mit F, Cl, Br, -CN, $-NR^9R^{10}$, $-OR^9$, $-SR^9$ oder $-COOR^9$ substituiertes $C_1$-$C_{18}$ Alkyl$\{X\}_p$ einschliesslich Methyl und Methoxy, Phenyl$\{X\}_p$,($C_1$-$C_6$-Alkyl)phenyl$\{X\}_p$, Benzyl$\{X\}_p$, ($C_1$-$C_6$-Alkyl)benzyl$\{X\}_p$;
unsubstituiertes $C_3$-$C_{12}$-Alkenoxy oder $C_3$-$C_6$-Alkinoxy, deren Alken- bzw. Alkingruppe nicht an das O-Atom gebunden ist; oder -F, -Cl, -Br, $-NO_2$, $-CF_3$, -CN, $-NR^9R^{10}$, $-COOR^9$, $-CONR^9R^{10}$, $-Si(CH_3)_3$, $-O\{C_2H_4-O\}_nR^{11}$ darstellt; $R^{11}$ H oder $C_1$-$C_4$-Alkyl ist;
X -O-, -SO- oder $-SO_2$-, p 1 und n 2 bis 20 darstellen; und
$R^9$ und $R^{10}$ je für H, $C_1$-$C_6$-Alkyl, oder $R^9$ und $R^{10}$ zusammen Tetra- oder Pentamethylen, 3-Oxapentylen oder $-CH_2CH_2N(C_1$-$C_6$-Alkyl$)CH_2CH_2$- stehen.

**9.** Verbindungen gemäss Anspruch 8, worin $R^1$ H ist.

**10.** Verbindungen gemäss Anspruch 1, worin $R^6$ bis $R^7$ H sind;
$R^1$ und $R^4$ unabhängig voneinander H oder einen Rest aus der Gruppe $-NO_2$, $-CF_3$, -CN, $-COOR^9$, $-CONR^9R^{10}$, $-Si(CH_3)_3$, $-S\{C_2H_4-O\}_nR^{11}$, $-O\{C_2H_4-O\}_nR^{11}$ bedeuten, wobei $R^9$ und $R^{10}$ je für H, $C_1$-$C_6$-Alkyl, und $R^9$ und $R^{10}$ zusammen Tetramethylen, Pentamethylen oder 3-Oxapentylen stehen; $R^{11}$ H oder $C_1$-$C_4$-Alkyl ist;
$R^2$ und $R^3$ unabhängig voneinander H oder einen Rest aus der Gruppe $C_5$-$C_{18}$-Alkyl, $C_3$-$C_{18}$-Alkoxy, $C_1$-$C_{18}$-Alkyl-$X^1$-, Phenyl-X-, ($C_1$-$C_6$-Alkyl)phenyl-X-, Benzyl$\{X\}_p$ oder ($C_1$-$C_6$-Alkyl)benzyl$\{X\}_p$, die unsubstituiert
oder mit -F, -Cl, -Br, -CN, $-NR^9R^{10}$, $-OR^9$, $-SR^9$ oder $-COOR^9$ substituiert sind,
oder unsubstituiertes $C_3$-$C_{12}$-Alkenoxy oder $C_3$-$C_{12}$-Alkenoxy, deren Alken-bzw. Alkingruppe nicht an das O-Atom gebunden ist; oder mit -F, -Cl, -Br, -CN, $-NR^9R^{10}$, $-SR^9$ oder $-OR^9$ substituiertes $C_1$-$C_4$-Alkyl oder $C_1$-$C_2$-Alkoxy; oder -F, -Cl, -Br, $-NO_2$, $-CF_3$, -CN, $-NR^9R^{10}$, $-Si(CH_3)_3$, oder $-\{OC_2H_4-O\}_nR^{11}$;
oder eines von $R^2$ und $R^3$ -F, -Cl, $-COOR^9$ oder $-CONR^9R^{10}$ sind und das andere von $R^2$ und $R^3$ ein wie in Anspruch 8 für $R^6$ definierter Rest ist, oder das andere von $R^2$ und $R^3$ H ist, wenn $R^1$ ein Rest ist; oder $R^2$ und $R^3$ $-COOR^9$, $-CONR^9R^{10}$, oder zusammen -CO-O-CO- oder $-CO-NR^9-CO$- sind, wenn $R^1$ die zuvor als Rest angegebenen Bedeutungen hat; oder $R^2$ und $R^3$ unabhängig Methoxy oder Ethoxy sind;
$R^9$ und $R^{10}$ je für H, $C_1$-$C_6$-Alkyl oder $R^9$ und $R^{10}$ zusammen für Tetramethylen, Pentamethylen, 3-Oxapentylen oder $-CH_2CH_2N(C_1$-$C_6$-Alkyl$)CH_2CH_2$-stehen, $R^{11}$ H oder $C_1$-$C_4$-Alkyl ist und n 2 bis 12 ist.

**11.** Verbindungen gemäss Anspruch 10, worin $R^4$ H ist und $R^1$ $-CF_3$ oder -COO($C_1$-$C_4$-Alkyl) bedeutet.

**12.** Verbindungen gemäss Anspruch 10, worin $R^1$ und $R^4$ H bedeuten.

**13.** Verbindungen gemäss Anspruch 1, worin $R^6$ und $R^7$ H sind und $R^1$ unsubstituiertes $C_1$-$C_{12}$-Alkylthio, $C_1$-$C_{12}$-Alkoxy, Phenyloxy, ($C_1$-$C_6$-Alkyl)phenyloxy oder ($C_1$-$C_6$-Alkyl)phenylthio oder mit -F, -Cl, -OH, $C_1$-$C_4$-Alkoxy oder $C_1$-$C_4$-Alkylthio substituiertes Phenylthio oder ($C_1$-$C_6$-Alkyl)phenylthio darstellt.

**14.** Verbindungen gemäss Anspruch 1, worin $R^2$ und $R^3$ als substituiertes Alkyl mit -CN oder -COO($C_1$-$C_{12}$-Alkyl) substituiertes $C_1$-$C_6$-Alkyl sind.

**15.** Verbindungen gemäss Anspruch 1, worin $R^1$ H, $-NO_2$, $-CF_3$ oder -COO($C_1$-$C_4$-Alkyl) ist; $R^4$ H bedeutet; $R^3$, $R^6$ und $R^7$ H sind und $R^2$ für $C_3$ bis $C_{18}$-Alkoxy, $C_3$-$C_{12}$-Hydroxyalkyl, $C_3$-$C_6$-Dihydroxyalkyl; $C_3$-$C_{12}$-Alkenoxy oder $C_3$-$C_6$-Alkinoxy, bei dem das -O-Atom nicht an die Alken- bzw. Alkingruppe gebunden ist, $-O\{CH_2CH_2-O\}_nR^{11}$ mit n = 2 bis 12 und $R^{11}$ gleich H oder $C_1$-$C_6$-Alkyl; unsubstituiertes oder mit -F, -Cl, $C_1$-$C_4$-Alkyl, $C_1$-$C_4$-Alkoxy oder $C_1$-$C_4$-Alkylthio substituiertes Phenyloxy; $-NR^9R^{10}$ mit $R^9$ und $R^{10}$ gleich $C_1$-$C_6$-Alkyl, oder zusammen Pentamethylen oder 3-Oxapentylen; $C_1$-$C_6$-Alkyl-SO-, $C_1$-$C_6$-Alkyl-$SO_2$-, Phenyl-SO- oder Phenyl-$SO_2$-; mit -CN oder -COO($C_1$-$C_6$-Alkyl) substituiertes $C_1$-$C_4$-Alkyl; oder -CN oder $-CF_3$ darstellt;
$R^2$ und $R^3$ unabhängig $C_1$-$C_2$-Alkoxy, $\{O-CH_2CH_2)-_nOR^{11}$ mit $R^{11}$ gleich H oder $C_1$-$C_6$-Alkyl und n gleich 2 bis 12; oder -F, -Cl, -Br darstellen oder die zuvor für $R^2$ angegebenen Bedeutungen haben; sowie $R^6$ und $R^7$ unabhängig H, -F, -Cl, -Br, -CN, $-CF_3$, $-Si(CH_3)_3$, $-NO_2$, $C_1$-$C_{12}$-Alkoxy oder -COO($C_1$-$C_6$-Alkyl) bedeuten, mit Ausnahme von $R^2$ und $R^6$ oder $R^7$ gleich -F und $R^1$ und $R^4$ gleich H; oder $R^1$

bis $R^4$ H sind, $R^6$ oder $R^7$ H und $R^7$ oder $R^6$, oder $R^6$ und $R^7$ unabhängig voneinander -F, -Cl, -Br, -CN, $-NO_2$, $-CF_3$, $-Si(CH_3)_3$, $-NO_2$, -COO($C_1$-$C_4$-Alkyl) oder $C_1$-$C_{12}$-Alkoxy darstellen.

**16.** Verfahren zur Herstellung von Verbindungen der Formel I, bei dem man ein Naphthalindicarbonsäureanhydrid der Formel II

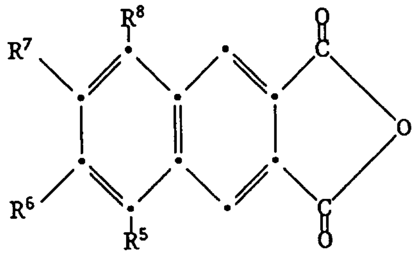

(II)

in Gegenwart einer Lewissäure mit einem Benzol der Formel III

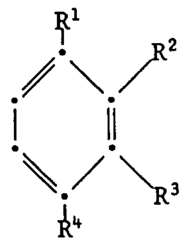

(III)

umsetzt, wobei $R^1$ bis $R^8$ die in Anspruch 1 angegebenen Bedeutungen haben, und Verbindungen der Formel I, worin mindestens eines von $R^1$ bis $R^4$, $R^6$ und $R^7$ $-NO_2$ oder Halogen bedeuten, gegebenenfalls mit einer nukleophilen Verbindung substituiert.

**17.** Durch Strahlung polymerisierbare Zusammensetzung, enthaltend (a) mindestens eine nichtflüchtige, monomere, oligomere oder polymere Verbindung mit mindestens einer photopolymerisierbaren oder photodimerisierbaren ethylenisch ungesättigten Doppelbindung und (b) mindestens eine Verbindung der Formel Ia als Photoinitiator oder Sensibilisator,

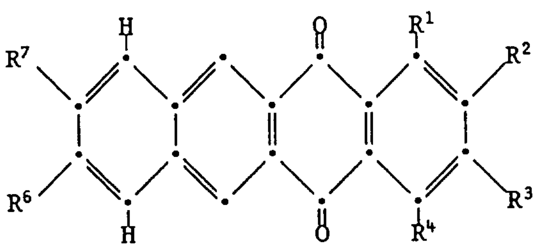

(Ia),

worin mindestens eines von $R^1$ bis $R^4$, $R^6$ und $R^7$ einen Substituenten darstellen, $R^1$ und $R^4$ unabhängig voneinander H, $C_1$-$C_{18}$-Alkoxy, $-O(C_mH_{2m}-O)_nR^{11}$ mit m gleich 2 bis 4, n gleich 1 bis 20 und $R^{11}$ gleich H oder $C_1$-$C_{12}$-Alkyl, $R^2$, $R^3$, $R^6$ und $R^7$ unabhängig voneinander H $C_1$-$C_{18}$-Alkoxy, $C_1$-$C_{18}$-Alkyl-SO-, $-O(C_mH_{2m}-O)_nR^{11}$, -COO($C_1$-$C_{18}$-Alkyl), $-COO(C_mH_{2m}-O)_nR^{11}$ oder mit 1 oder 2 -COO-($C_1$-$C_{18}$-Alkyl) oder $-COO(C_mH_{2m}-O)_nR^{11}$ substituiertes $C_1$-$C_6$-Alkyl $C_1$-$C_6$-Alkoxy darstellen, wobei $R^{11}$, m und n die in Anspruch 1 angegebenen Bedeutungen haben.

**18.** Zusammensetzungen gemäss Anspruch 17, worin die Verbindung der Formel Ia in einer Menge von 0,01 bis 20 Gew.-% einverleibt ist, bezogen auf die photopolymerisierbare oder photodimerisierbare Verbindung.

**19.** Beschichtetes Substrat, das auf mindestens einer Oberfläche mit einer Zusammensetzung gemäss Anspruch 17 beschichtet ist.

## Claims

**1.** A compound of the formula I

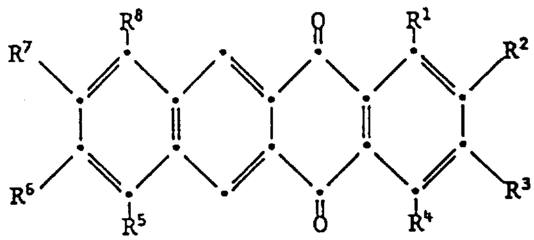

(I),

wherein $R^5$ and $R^8$ are H and

a) $R^1$, $R^2$, $R^3$ and $R^4$ are H and $R^6$ and $R^7$ are each H and at least one of the radicals $R^6$ and $R^7$, independently of one another, is a radical from the group comprising $C_1$-$C_{20}$alkyl-(-X-)-$_p$, with the exception of methoxy, $C_2$-$C_{18}$alkenyl-(-X-)-$_p$, $C_2$-$C_{18}$alkynyl-(-X-)-$_p$, $C_3$-$C_8$cycloalkyl-(-X-)-$_p$,($C_1$-$C_{12}$alkyl)-$C_3$-$C_8$cycloalkyl-(-X-)-$_p$, $C_3$-$C_8$cycloalkyl-$C_rH_{2r}$-(-X-)-$_p$, ($C_1$-$C_{12}$alkyl) -$C_3$-$C_8$cycloalkyl-$C_rH_{2r}$-(-X-)-$_p$, phenyl-(-X-)-$_p$, ($C_1$-$C_{12}$alkyl)phenyl-(-X-)-$_p$, phenyl-$C_rH_{2r}$-(-X-)-$_p$, and ($C_1$-$C_{12}$alkyl)phenyl-$C_rH_{2r}$-(-X-)-$_p$ which are unsubstituted or substituted by halogen, -CN, furfuryl, -$NR^9R^{10}$, -$OR^9$, -SR' or -$COOR^9$, r is 1 or 2, p is 1 and X is -O-, -SO- or -$SO_2$-, or $R^6$ and $R^7$ independently are a radical from the group comprising halogen,
-$NO_2$, -$CF_3$, -CN, -$NR^9R^{10}$, -$COOR^9$, -$CONR^9R^{10}$, -COCl, -SH, -Si($C_1$-$C_4$alkyl)$_3$ and -O-($C_mH_{2m}$-O)-$_nR^{11}$, or $R^6$ and $R^7$ together are -CO-O-CO- or -CO-$NR^9$-CO-, $R^9$ and $R^{10}$ independently of one another are H, $C_1$-$C_{12}$-alkyl, phenyl or (-$C_mH_{2m}$-O)$_qR^{11}$, or $R^9$ and $R^{10}$ together are tetramethylene, pentamethylene, 3-oxapentylene or -$CH_2CH_2NR^9CH_2CH_2$-, $R^{11}$ is H or $C_1$-$C_{12}$alkyl, m is a number from 2 to 4, n is a number from 2 to 20 and q is a number from 1 to 20, or

b) $R^1$ to $R^4$, $R^6$ and $R^7$ independently of one another are H or one of the radicals defined above, including methoxy, wherein $R^1$ and $R^4$ are also radicals with X in the meaning of -S- and at least one of $R^1$ to $R^4$ and $R^6$ and $R^7$ is a radical, with the exception of $R^2$ and $R^6$ or $R^7$ as -F, or

c) $R^6$ and $R^7$ are H, at least one of $R^1$, $R^2$, $R^3$ and $R^4$ is a radical and $R^1$ and $R^4$ independently of one another are H or a radical from the group comprising -$NO_2$, -$CF_3$, -CN, -$COOR^9$, -$CONR^9R^{10}$, -COCl, -Si($C_1$-$C_4$alkyl)$_3$, -S($C_mH_{2m}$-O)$_nR^{11}$ and -O(-$C_mH_{2m}$-O)$_nR^{11}$, and $C_1$-$C_{20}$alkyl-$X^1$-, which is unsubstituted or substituted as defined under a); $C_2$-$C_{18}$alkenyl-(-$X^1$-)-$_p$, $C_2$-$C_{18}$alkynyl-(-$X^1$-)-$_p$, $C_3$-$C_8$cycloalkyl-(-$X^1$-)-$_p$, ($C_1$-$C_{12}$alkyl)$C_3$-$C_8$cycloalkyl-(-$X^1$-)-$_p$, $C_3$-$C_8$cycloalkyl-$C_rH_{2r}$-(-$X^1$-)-$_p$, or ($C_1$-$C_{12}$alkyl)-$C_3$-$C_8$cycloalkyl-$C_rH_{2r}$-(-$X^1$-)-$_p$; phenyl-(- $X^1$-)-$_p$ ($C_1$-$C_{12}$alkyl)phenyl-(-$X^1$-)-$_p$; phenyl-$C_rH_{2r}$-(-X-)-$_p$ and ($C_1$-$C_{12}$alkyl)phenyl-$C_rH_{2r}$-(-X-)-$_p$;and $C_1$-$C_{20}$alkylthio which is substituted as defined above under a), phenyloxy, ($C_1$-$C_{12}$alkyl)phenyloxy and $C_1$-$C_{20}$alkoxy which is substituted by halogen, -CN, -$NR^9R^{10}$, -$SR^9$, -$OR^9$ or $COOR^9$; wherein $R^1$ is not -COOH if $R^2$, $R^3$ and $R^4$ are H, and $R^2$ and $R^3$ independently of one another are H or a radical from the group comprising $C_5$-$C_{20}$alkyl, $C_3$-$C_{20}$alkoxy and $C_1$-$C_{20}$alkyl-$X^1$-, which are unsubstituted or substituted as defined above under a); and phenyl-X-, ($C_1$-$C_{12}$alkyl)phenyl-(-X-)-$_p$,($C_1$-$C_{12}$alkyl)phenyl-$C_rH_{2r}$-(-X-)-$_p$, phenyl-$C_rH_{2r}$-(-X-)-$_p$, $C_2$-$C_{20}$alkenyl-(-X-)-$_p$, $C_2$-$C_{20}$alkynyl-(-X-)-$_p$, $C_3$-$C_8$cycloalkyl-(-X-)-$_p$,($C_1$-$C_{12}$alkyl)-$C_3$-$C_8$cycloalkyl-(-X-)-$_p$, $C_3$-$C_8$cycloalkyl-$C_rH_{2r}$-(-X-)-$_p$, or ($C_1$-$C_{12}$alkyl)-$C_3$-$C_8$cycloalkyl-$C_rH_{2r}$-(-X-)-$_p$; or one of $R^2$ and $R^3$ or $R^2$ and $R^3$ are a radical from the group comprising $C_1$-$C_4$alkyl and $C_1$-$C_2$alkoxy, which are substituted by halogen, -CN, -$NR^9R^{10}$, -$OR^9$, $SR^9$ or -$COOR^9$, with the exception of 2-hydroxyethyl, or one of $R^2$ and $R^3$ or $R^2$ and $R^3$ are a radical from the group comprising SH, -$NO_2$, - $CF_3$, -CN, -$NR^9R^{10}$, -Si($C_1$-$C_4$alkyl)$_3$ and -O($C_mH_{2m}$-O)$_nR^{11}$, or one of $R^2$ and $R^3$ are halogen, -$COOR^9$, -$CONR^9R^{10}$ or -COCl and the other of $R^2$ and $R^3$ is a radical as defined under a), or the other of $R^2$ and $R^3$ is H, if at least one of $R^1$ and $R^4$ is one of the radicals defined above; or $R^2$ and $R^3$ together are -CO-O-CO- or -CO-$NR^9$-CO- and at least one of $R^1$ and $R^4$ are one of the radicals defined above, or $R^2$ and $R^3$ together independently of one another are $C_1$-$C_2$alkoxy, wherein $R^9$, $R^{10}$, $R^{11}$, X, m, n, p, q, r and s are as defined under a) and $X^1$ is -SO- or -$SO_2$-or

d) $R^6$ and $R^7$ are H and $R^1$ is unsubstituted $C_1$-$C_{20}$alkylthio, $C_1$-$C_{20}$-alkoxy, phenyloxy, phenylthio, $C_1$-$C_{12}$alkylphenyloxy or $C_1$-$C_{12}$alkylphenylthio, or phenylthio or $C_1$-$C_{12}$alkylphenylthio which is substituted as defined under a).

**2.** A compound according to claim 1, in which $R^4$, or $R^1$ and $R^4$ are H.

**3.** A compound according to claim 1, in which $R^2$ or $R^3$, or $R^2$ and $R^3$, are a radical.

**4.** A compound according to claim 1, in which $R^1$, $R^2$ and $R^3$ or $R^1$ and $R^2$ or $R^1$ and $R^3$ are a radical and $R^4$ is H.

**5.** A compound according to either of claims 3 and 4, in which $R^6$ or $R^7$ or $R^6$ and $R^7$ are H or a radical.

**6.** A compound according to claim 1, in which $R^1$ to $R^4$ are H; at least one of the radicals $R^6$ or $R^7$ is a radical from the group comprising $C_1$-$C_{18}$alkyl-(-X-)-$_p$, phenyl-(-X-)-$_p$, ($C_1$-$C_6$alkyl)phenyl-(-X-)-$_p$, benzyl-(-X-)-$_p$ and ($C_1$-$C_6$alkyl)benzyl-(-X-)-$_p$, which are unsubstituted or substituted by F, Cl, Br, -CN, -$NR^9R^{10}$, -$OR^9$, -$SR^9$ or -$COOR^9$; unsubstituted $C_3$-$C_{12}$alkenoxy and $C_3$-$C_6$alkynoxy, the alkene or alkyne group of which is not bonded to the O atom; or -F, -Cl, -Br, -$NO_2$, - $CF_3$, -CN, -$NR^9R^{10}$, $COOR^9$, -$CONR^9R^{10}$, -$Si(CH_3)_3$ or -$O(C_2H_4O\text{-})_nR^{11}$; $R^{11}$ is H or $C_1$-$C_4$alkyl; X is -O-, -SO- or -$SO_2$-, p is 1 and n is 2 to 20, and in which $R^9$ and $R^{10}$ are each H or $C_1$-$C_6$alkyl, or $R^9$ and $R^{10}$ together are tetra- or pentamethylene, 3-oxapentylene or -$CH_2CH_2N(C_1$-$C_6$alkyl$)CH_2CH_2$.

**7.** A compound according to claim 6, in which $R^6$ or $R^7$ or $R^6$ and $R^7$ are -F, -Cl, -Br, -CN, -$NO_2$, -COOH, -COO($C_1$-$C_4$)alkyl, -$CF_3$, -$Si(CH_3)_3$ or unsubstituted or substituted $C_1$-$C_{12}$alkyl-X-.

**8.** A compound according to claim 1, in which $R^4$ is H; at least one of the radicals $R^1$, $R^2$ and $R^3$ and at least one of the radicals $R^6$ and $R^7$ are a radical from the group comprising $C_1$-$C_{18}$alkyl-(-X-)-$_p$, including methyl and methoxy, phenyl-(-X-)-$_p$, ($C_1$-$C_6$alkyl)phenyl-(-X-)-$_p$,benzyl-(-X-)-$_p$ and ($C_1$-$C_6$alkyl)-benzyl-(-X-)-$_p$, which are unsubstituted or substituted by F, Cl, Br, -CN, -$NR^9R^{10}$, -$OR^9$, -$SR^9$ or -$COOR^9$; unsubstituted $C_3$-$C_{12}$alkenoxy or $C_3$-$C_6$alkynoxy, the alkene or alkyne group of which is not bonded to the O atom; or -F, -Cl, -Br, -$NO_2$, - $CF_3$, -CN, -$NR^9R^{10}$, -$COOR^9$, -$CONR^9R^{10}$, -$Si(CH_3)_3$ and -O-($C_2H_4$O-)-$_nR^{11}$; $R^{11}$ is H or
$C_1$-$C_4$alkyl; X is -O-, -SO- or -$SO_2$-, p is 1 and n is 2 to 20; and $R^9$ and $R^{10}$ are each H or $C_1$-$C_6$alkyl, or $R^9$ and $R^{10}$ together are tetra- or pentamethylene, 3-oxapentylene or -$CH_2CH_2N(C_1$-$C_6$alkyl$)CH_2CH_2$-.

**9.** A compound according to claim 8, in which $R^1$ is H.

**10.** A compound according to claim 1, in which $R^6$ to $R^7$ are H; $R^1$ and $R^4$ independently of one another are H or a radical from the group comprising -$NO_2$, -$CF_3$, -CN, -$COOR^9$, -$CONR^9R^{10}$, -$Si(CH_3)_3$, -S-($C_2H_4$-O)$_nR^{11}$ and -O($C_2H_4$-O)$_nR^{11}$, in which $R^9$ and $R^{10}$ are each H or $C_1$-$C_6$alkyl, and $R^9$ and $R^{10}$ together are tetramethylene, pentamethylene or 3-oxapentylene; $R^{11}$ is H or $C_1$-$C_4$alkyl; $R^2$ and $R^3$ independently of one another are H or a radical from the group comprising $C_5$-$C_{18}$alkyl, $C_3$-$C_{18}$alkoxy, $C_1$-$C_{18}$alkyl-$X^1$-, phenyl-X-, ($C_1$-$C_6$alkyl)phenyl-X-, benzyl-(-X-)-$_p$, or ($C_1$-$C_6$alkyl)benzyl-(-X-)-$_p$ which are unsubstituted or substituted by - F, -Cl, -Br, -CN, -$NR^9R^{10}$, -$OR^9$, -$SR^9$ or -$COOR^9$, or unsubstituted $C_3$-$C_{12}$alkenoxy and $C_3$-$C_{12}$alkynoxy, the alkene or alkyne group of which is not bonded to the O atom; or $C_1$-$C_4$alkyl or $C_1$-$C_2$alkoxy which are substituted by -F, -Cl, -Br, -CN, -$NR^9R^{10}$, -$SR^9$ or -$OR^9$; or -F, -Cl, - Br, -$NO_2$, -$CF_3$, -CN, -$NR^9R^{10}$, -$Si(CH_3)_3$ or -(-$OC_2H_4$O-)-$_nR^{11}$; or one of $R^2$ and $R^3$ is -F, -Cl, -$COOR^9$ or -$CONR^9R^{10}$ and the other of $R^2$ and $R^3$ is a radical as defined for $R^6$ in claim 8, or the other of $R^2$ and $R^3$ is H, if $R^1$ is a radical; or $R^2$ and $R^3$ are -$COOR^9$ or -$CONR^9R^{10}$, or together are -CO-O-CO- or -CO-$NR^9$-CO-, if $R^1$ is a radical as defined above; or $R^2$ and $R^3$ independently are methoxy or ethoxy; or $R^9$ and $R^{10}$ are each H or $C_1$-$C_6$alkyl, or $R^9$ and $R^{10}$ together are tetramethylene, pentamethylene, 3-oxapentylene or -$CH_2CH_2N(C_1$-$C_6$alkyl$)CH_2CH_2$-, $R^{11}$ is H or $C_1$-$C_4$alkyl and n is 2 to 12.

**11.** A compound according to claim 10, in which $R^4$ is H and $R^1$ is -$CF_3$ or -COO($C_1$-$C_4$alkyl).

**12.** A compound according to claim 10, in which $R^1$ and $R^4$ are H.

**13.** A compound according to claim 1, in which $R^6$ and $R^7$ are H and $R^1$ is unsubstituted $C_1$-$C_{12}$alkylthio, $C_1$-$C_{12}$alkoxy, phenyloxy, ($C_1$-$C_6$alkyl)phenyloxy or ($C_1$-$C_6$alkyl)phenylthio, or phenylthio or ($C_1$-$C_6$alkyl)phenylthio which is substituted by -F, -Cl, -OH, $C_1$-$C_4$alkoxy or $C_1$-$C_4$alkylthio.

**14.** A compound according to claim 1, in which $R^2$ and $R^3$ as substituted alkyl are $C_1$-$C_6$alkyl which is substituted by -CN or -COO($C_1$-$C_{12}$alkyl).

**15.** A compound according to claim 1, in which $R^1$ is H, $-NO_2$, $-CF_3$ or -COO($C_1$-$C_4$alkyl); $R^4$ is H; $R^3$, $R^6$ and $R^7$ are H and $R^2$ is $C_3$-$C_{18}$alkoxy, $C_3$-$C_{12}$hydroxyalkyl or $C_3$-$C_6$dihydroxyalkyl; $C_3$-$C_{12}$alkenoxy or $C_3$-$C_6$alkynoxy, in which the -O- atom is not bonded to the alkene or alkyne group, or $-O(CH_2CH_2-O)-_nR^{11}$ where n is 2 to 12 and $R^{11}$ is H or $C_1$-$C_6$alkyl; phenyloxy which is unsubstituted or substituted by -F, -Cl, $C_1$-$C_4$alkyl, $C_1$-$C_4$alkoxy or $C_1$-$C_4$alkylthio; $-NR^9R^{10}$, where $R^9$ and $R^{10}$ are $C_1$-$C_6$alkyl, or together are pentamethylene or 3-oxapentylene; $C_1$-$C_6$alkyl-SO-, $C_1$-$C_6$alkyl-$SO_2$-, phenyl-SO- or phenyl-$SO_2$-; $C_1$-$C_4$alkyl which is substituted by -CN or COO($C_1$-$C_6$alkyl); or -CN or $-CF_3$; $R^2$ and $R^3$ independently of one another are $C_1$-$C_2$alkoxy or $-(O-CH_2CH_2)_nOR^{11}$, where $R^{11}$ is H or $C_1$-$C_6$alkyl and n is 2 to 12; or -F, -Cl or -Br, or are as defined above for $R^2$; and $R^6$ and $R^7$ independently are H, -F, -Cl, -Br, -CN, $-CF_3$, $-Si(CH_3)_3$, $-NO_2$, $C_1$-$C_{12}$alkoxy or -COO($C_1$-$C_6$alkyl), with the exception of $R^2$ and $R^6$ or $R^7$ as -F and $R^1$ and $R^4$ as H; or $R^1$ to $R^4$ are H, $R^6$ or $R^7$ is H and $R^7$ or $R^6$ or $R^6$ and $R^7$ independently of one another are -F, -Cl, -Br, -CN, $-NO_2$, $-CF_3$, -Si $(CH_3)_3$, $-NO_2$, -COO($C_1$-$C_4$alkyl) or $C_1$-$C_{12}$alkoxy.

**16.** A process for the preparation of a compound of the formula I, which comprises reacting a naphthalenedicarboxylic anhydride of the formula II

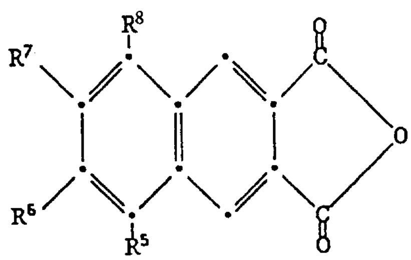

(II)

with a benzene of the formula III

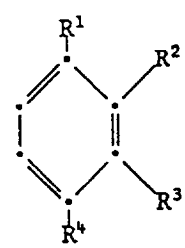

(III)

in which $R^1$ to $R^4$ are as defined in claim 1, in the presence of a Lewis acid and, if appropriate, substituting a compound of the formula I in which at least one of $R^1$ to $R^4$, $R^6$ and $R^7$ is $-NO_2$ or halogen with a nucleophilic compound.

**17.** A composition which can be polymerized by radiation and contains (a) at least one non-volatile, monomeric, oligomeric or polymeric compound having at least one photopolymerizable or photodimerizable ethylenically unsaturated double bond and (b) at least one compound of the formula Ia as a photoinitiator or sensitizer

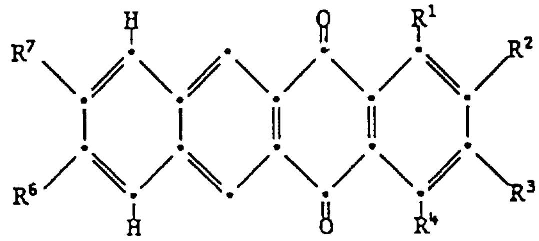

(Ia),

in which at least one of $R^1$ to $R^4$, $R^6$ and $R^7$ is a radical, $R^1$ and $R^4$ independently of one another are H, $C_1$-$C_{18}$alkoxy or -O($C_mH_{2m}$-O)$_n$$R^{11}$, where m is 2 to 4, n is 1 to 20 and $R^{11}$ is H or $C_1$-$C_{12}$alkyl, $R^2$, $R^3$, $R^6$ and $R^7$ independently of one another are H, $C_1$-$C_{18}$alkoxy, $C_1$-$C_{18}$alkyl-SO-, -O-($C_mH_{2m}$-O-)-$_n$$R^{11}$, -COO($C_1$-$C_{18}$alkyl), -COO($C_mH_{2m}$-O)$_n$$R^{11}$ or $C_1$-$C_6$alkyl or $C_1$-$C_6$alkoxy which is substituted by one or two -COO($C_1$-$C_{18}$alkyl) or
-COO($C_mH_{2m}$-O)$_n$$R^{11}$, wherein $R^{11}$, m and n are as in claim 1.

**18.** A composition according to claim 17, in which the compound of the formula Ia is incorporated in an amount of 0.01 to 20 % by weight, based on the photopolymerizable or photodimerizable compound.

**19.** A coated substrate which is coated on at least one surface with a composition according to claim 17.

**Revendications**

**1.** Composés de formule I :

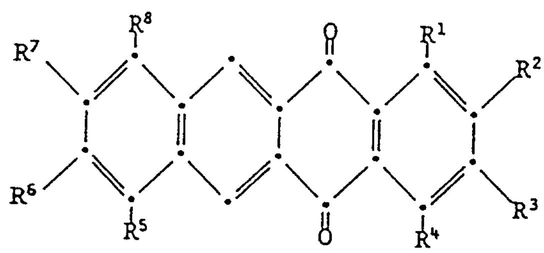

(I)

dans laquelle $R^5$ et $R^8$ sont H et

a) $R^1$, $R^2$, $R^3$, $R^4$ sont H, et

$R^6$ et $R^7$ représente chacun H et au moins l'un des radicaux $R^6$ et $R^7$ indépendamment l'un de l'autre représentent un radical pris dans le groupe des suivants:
un (alkyle en $C_1$-$C_{12}$)-phényl-$C_rH_{2r}$$(X)_p$,
un phényl-$C_rH_{2r}$$(X)_p$,
un (alkyle en $C_1$-$C_{12}$)-cycloalkyle en $C_3$-$C_8$-$C_rH_{2r}$$(X)_p$, cycloalkyle en $C_3$-$C_8$-$C_rH_{2r}$$(X)_p$,
un (alkyle en $C_1$-$C_{12}$)-(cycloalkyle en $C_3$-$C_8$)$(X)_p$,
un (cycloalkyle en $C_3$-$C_8$)$(X)_p$,
un (alcynyle en $C_2$-$C_{18}$)$(X)_p$,
un (alcényle en $C_2$-$C_{18}$)$(X)_p$,
un (alkyle en $C_1$-$C_{20}$)$(X)_p$, à l'exception du méthoxy,
non substitué ou substitué par un halogène, -CN, un furfuryle, -$NR^9R^{10}$, -$OR^9$, -$SR^9$ ou -$COOR^9$,
r correspond à 1 ou 2, p à 1 et X signifie -O-, -SO- ou -$SO_2$-, ou $R^6$, $R^7$ signifient indépendamment l'un de l'autre un radical pris dans le groupe :
un halogène, -$NO_2$, -$CF_3$, -CN, -$NR^9R^{10}$, -$COOR^9$, -$CONR^9R^{10}$, -COCl, -SH, -Si(alkyle en $C_1$-$C_4$)$_3$ ou -O$(C_mH_{2m}$-O$)_q$$R^{11}$, ou
$R^6$ et $R^7$ ensemble représentent -CO-O-CO- ou -CO-$NR^9$-CO-,
$R^9$ et $R^{10}$ représentent indépendamment l'un de l'autre H, un alkyle en $C_1$-$C_{12}$, un phényle ou $(C_mH_{2m}$-O$)_q$$R^{11}$, ou $R^9$ et $R^{10}$ ensemble représentent le tétraméthylène, le pentaméthylène, le 3-oxapentylène ou -$CH_2CH_2NR^9CH_2CH_2$-, $R^{11}$ est H ou un alkyle en $C_1$-$C_{12}$,
m vaut de 2 à 4, n vaut de 2 à 20 et q vaut de 1 à 20, ou

b) $R^1$ à $R^4$, $R^6$ et $R^7$ représentent indépendamment l'un de l'autre représentent H ou l'un des radicaux définis auparavant, y compris le méthoxy, $R^1$ et $R^4$ étant aussi des radicaux avec X ayant la signification de -S- et au moins un de $R^1$ à $R^4$ ainsi que $R^6$ et $R^7$ signifient un radical, à l'exception de $R^2$ et $R^6$ ou $R^7$ représentant F, ou

c) $R^6$ et $R^7$ correspondent à H, au moins un des radicaux $R^1$, $R^2$, $R^3$ et $R^4$ signifie un radical, et $R^1$ et $R^4$ indépendamment l'un de l'autre signifient H ou un radical pris dans le groupe $-NO_2$, $-CF_3$, -CN, $-COOR^9$, $-CONR^9R^{10}$, -COCl, -Si(alkyle en $C_1$-$C_4$)$_3$, $-S\{C_mH_{2m}-O\}_nR^{11}$, $-O\{C_mH_{2m}-O\}_nR^{11}$,
un (alkyle en $C_1$-$C_{12}$)-phényl-$C_rH_{2r}\{X\}_p$ ;
un phényl-$C_rH_{2r}\{X\}_p$,
un phényl$\{X^1\}_p$ ou un (alkyle en $C_1$-$C_{12}$)-phényl-$\{X^1\}_p$,
un (alkyle en $C_1$-$C_{12}$)-cycloalkyle en $C_3$-$C_8$-$C_rH_{2r}$-$\{X^1\}_p$,
un (cycloalkyle en $C_3$-$C_8$)-$C_rH_{2r}\{X'\}_p$,
un (alkyle en $C_1$-$C_{12}$)-cycloalkyle en $C_3$-$C_8$-$\{X^1\}_p$,
un cycloalkyle en $C_3$-$C_8$-$\{X^1\}_p$,
un (alcynyle en $C_2$-$C_{18}$)-(-$X^1$-$_p$,
un (alcényle en $C_2$-$C_{18}$)$\{X^1\}_p$,
un alkyle en $C_1$-$C_{20}$-$X^1$-
non substitué ou substitué comme défini auparavant au point a) ;
un (alkyle en $C_1$-$C_{12}$)-phényloxy, un phényloxy ou un alkylthio en $C_1$-$C_{20}$ substitué comme défini auparavant au point a) ou un alcoxy en $C_1$-$C_{20}$ substitué par un halogène, -CN, $-NR^9R^{10}$, $-SR^9$, $-OR^9$ ou $-COOR^9$ ; où $R^1$ n'est pas -COOH, lorsque $R^2$, $R^3$ et $R^4$ signifient H, et
$R^2$ et $R^3$ indépendamment l'un de l'autre signifient H ou un radical pris dans le groupe suivant :
un (alkyle en $C_1$-$C_{12}$)-cycloalkyle en $C_3$-$C_8$-$C_rH_{2r}$-$\{X\}_p$, un (cycloalkyle en $C_3$-$C_8$)-$C_rH_{2r}\{X\}_p$ un (alkyle en $C_1$-$C_{12}$)-cycloalkyle en $C_3$-$C_8\{X\}_p$, un (cycloalkyle en $C_3$-$C_8$)$\{X\}_p$, un (alcynyle en $C_2$-$C_{20}$)$\{X\}_p$, un (alcényle en $C_2$-$C_{20}$)$\{X\}_p$, un phényl-$C_rH_{2r}\{X\}_p$, un (alkyle en $C_1$-$C_{12}$)-phényl-$C_rH_{2r}\{X\}_p$, un (alkyle en $C_1$-$C_{12}$)-phényl$\{X\}_p$,
un phényl-X-, un (alkyle en $C_1$-$C_{20}$)-$X^1$-, un alcoxy en $C_3$-$C_{20}$ ou un alkyle en $C_5$-$C_{20}$
non substitué ou substitué comme défini auparavant au point a) ; ou
un de $R^2$ et $R^{3,}$ ou $R^2$ et $R^{3,}$ signifient un radical pris dans le groupe un alcoxy en $C_1$-$C_2$ à l'exception de 2-hydroxyéthyle ou un alkyle en $C_1$-$C_4$ substitué par un halogène, -CN, $-NR^9R^{10}$, $-OR^9$, $-SR^9$, $-COOR^9$ ou un des radicaux $R^2$ ou $R^{3,}$ ou $R^2$ et $R^{3,}$ représentent un radical pris dans le groupe -SH, $-NO_2$, $-CF_3$, -CN, $-NR^9R^{10}$, -Si(alkyle en $C_1$-$C_4$)$_3$ ou $-O\{C_mH_{2m}-O\}_nR^{11}$ ;
ou un des radicaux $R^2$ et $R^3$ représente un halogène, $-COOR^9$, $-CONR^9R^{10}$ ou -COCl et l'autre de $R^2$ et $R^3$ un radical comme défini auparavant au point a), ou l'autre de $R^2$ et $R^3$ est H, lorsque au moins un des $R^1$ et $R^4$ représente un des radicaux définis auparavant ; ou
$R^2$ et $R^3$ ensemble avec -CO-O-CO- ou $-CO-NR^9-CO-$ et au moins l'un de $R^1$ et $R^4$ représente un radical tel que défini auparavant, ou
$R^2$ et $R^3$ ensemble, indépendamment l'un de l'autre, sont un alcoxy en $C_1$-$C_2$, où $R^9$, $R^{10}$, $R^{11}$, X, m, n, p, q, r et s ont les significations données au point a) et $X^1$ représente -SO- ou $-SO_2-$ ;

d) $R^6$ et $R^7$ signifient H et $R^1$ représente un alkylphénylthio en $C_1$-$C_{12}$, un alkylphényloxy en $C_1$-$C_{12}$, un phénylthio, un phényloxy, un alcoxy en $C_1$-$C_{20}$, un alkylthio en $C_1$-$C_{20}$ non substitué, ou un alkylphénylthio en $C_1$-$C_{12}$ ou un phénylthio substitué comme défini au point a).

2. Composés selon la revendication 1, où $R^4$, ou $R^1$ et $R^4$ représentent H.

3. Composés selon la revendication 1, où $R^2$ ou $R^3$, ou $R^2$ et $R^3$ représentent un radical.

4. Composés selon la revendication 1, où $R^1$, $R^2$ et $R^3$, ou $R^1$ et $R^2$, ou $R^1$ et $R^3$ représentent un radical et $R^4$ signifie H.

5. Composés selon les revendications 3 ou 4, où $R^6$ ou $R^7$, ou $R^6$ et $R^7$ signifient H ou un radical.

6. Composés selon la revendication 1, où $R^1$ à $R^4$ signifient H ; au moins l'un des radicaux $R^6$ ou $R^7$ représente un radical pris dans le groupe suivant :
un (alkyle en $C_1$-$C_6$)-benzyl$\{X\}_p$,
un benzyl$\{X\}_p$,
un (alkyle en $C_1$-$C_6$)-phényl$\{X\}_p$,
un phényl$\{X\}_p$,

un (alkyle en $C_1$-$C_{18}$)$\{X\}_p$ non substitué ou substitué par -F, -Cl, -Br, -CN, -$NR^9R^{10}$, -$OR^9$, -$SR^9$ ou -$COOR^9$ ; un alcénoxy en $C_3$-$C_{12}$ ou un alcynoxy en $C_3$-$C_6$ non substitué, dont le groupe alcène ou alcyne n'est pas lié à l'atome d'oxygène ; ou représente F, Cl, Br, -$NO_2$, -$CF_3$, -CN, -$NR^9R^{10}$, -$COOR^9$, -$CONR^9R^{10}$, -$Si(CH_3)_3$ ou -$O\{C_2H_4$-$O\}_nR^{11}$ ; $R^{11}$ est H ou un alkyle en $C_1$-$C_4$ ; X représente -O-, -SO- ou -$SO_2$-, p est 1 et n vaut de 2 à 20, et où $R^9$ et $R^{10}$ correspondant chacun à H, à un alkyle en $C_1$-$C_6$, ou $R^9$ et $R^{10}$ ensemble au tétraméthyle ou pentaméthylène, à 3-oxapentylène ou -$CH_2CH_2N$(alkyle en $C_1$-$C_6$)$CH_2CH_2$-.

**7.** Composés selon la revendication 6, où $R^6$ ou $R^{7}$, ou $R^6$ et $R^7$ signifient -F, -Cl, -Br, -CN, -$NO_2$, -COOH, -COO(alkyle en $C_1$-$C_4$), -$CF_3$, -$Si(CH_3)_3$ ou un (alkyle en $C_1$-$C_{12}$)-X- non substitué ou substitué.

**8.** Composés selon la revendication 1, où $R^4$ est H ; au moins un des radicaux $R^1$, $R^2$ et $R^3$ ainsi qu'au moins un des radicaux $R^6$ et $R^7$ représente un radical pris dans le groupe : un (alkyle en $C_1$-$C_6$)-benzyl$\{X\}_p$, un benzyl$\{X\}_p$, un (alkyle en $C_1$-$C_6$)-phényl$\{X\}_p$, un phényle$\{X\}_p$, un (alkyle en $C_1$-$C_{18}$)-$\{X\}_p$, y compris le méthyle et le méthoxy, non substitué ou substitué par -F, -Cl, -Br, -CN, -$NR^9R^{10}$, -$OR^9$, -$SR^9$ ou -$COOR^9$ ; un alcénoxy en $C_3$-$C_{12}$ ou un alcynoxy en $C_3$-$C_6$ non substitué, dont le groupe alcène ou alcyne n'est pas lié à l'atome d'oxygène ; ou représente F, Cl, Br, -$NO_2$, -$CF_3$, -CN, -$NR^9R^{10}$, -$COOR^9$, -$CONR^9R^{10}$, -$Si(CH_3)_3$ ou -$O\{C_2H_4O\}_nR^{11}$ ;
$R^{11}$ est H ou un alkyle en $C_1$-$C_4$ ;
X représente -O-, -SO- ou -$SO_2$-, p est 1 et n vaut de 2 à 20 ; et
$R^9$ et $R^{10}$ correspondent chacun à H, à un alkyle en $C_1$-$C_6$, ou $R^9$ et $R^{10}$ ensemble correspondent au tétraméthylène ou pentaméthylène, à 3-oxapentylène ou -$CH_2CH_2N$(alkyle en $C_1$-$C_6$)$CH_2CH_2$-.

**9.** Composés selon la revendication 8, où $R^1$ est H.

**10.** Composés selon la revendication 1, où $R^6$ à $R^7$ sont H ; $R^1$ et $R^4$ indépendamment l'un de l'autre signifient H ou un radical pris dans le groupe suivant : -$NO_2$, -$CF_3$, -CN, -$COOR^9$, -$CONR^9R^{10}$, -$Si(CH_3)_3$, -$S\{C_2H_4$-$O\}_nR^{11}$, -$O$-$\{C_2H_4$-$O\}_nR^{11}$, où $R^9$ et $R^{10}$ correspondent chacun à H, à un alkyle en $C_1$-$C_6$, et $R^9$ et $R^{10}$ ensemble au tétraméthylène, au pentaméthylène ou à 3-oxapentylène ;
$R^{11}$ est H ou un alkyle en $C_1$-$C_4$ ;
$R^2$ et $R^3$ indépendamment l'un de l'autre sont H ou un radical pris dans le groupe suivant : un alkyle en $C_5$-$C_{18}$,un alcoxy en $C_3$-$C_{18}$, un alkyle en $C_1$-$C_{18}$-$X^1$-, un phényl-X-, un (alkyle en $C_1$-$C_6$)-phényl-X-, un benzyl$\{X\}_p$ ou un (alkyle en $C_1$-$C_6$)-benzyl-$X_p$- qui sont non substitués ou substitués par F, Cl, Br, -CN, -$NR^9R^{10}$, -$OR^9$, -$SR^9$ ou -$COOR^9$, ou
un alcénoxy en $C_3$-$C_{12}$ ou un alcynoxy en $C_3$-$C_{12}$ non substitué, dont le groupe alcène ou alcyne n'est pas lié à l'atome d'oxygène ; ou un alcoxy en $C_1$-$C_2$ ou un alkyle en $C_1$-$C_4$ substitué par -F, -Cl, -Br, -CN, -$NR^9R^{10}$, -$SR^9$ ou -$OR^9$ ; ou -F, -Cl, -Br, -$NO_2$, -$CF_3$, -CN, -$NR^9R^{10}$, -$Si(CH_3)_3$ ou $\{O$-$C_2H_4O\}_nR^{11}$ ; ou un des radicaux $R^2$ et $R^3$ est -F, -Cl, -$COOR^9$ ou -$CONR^9R^{10}$ et l'autre de $R^2$ et $R^3$ est un radical tel que défini pour $R^6$ dans la revendication 8, ou l'autre de $R^2$ et $R^3$ est H, lorsque $R^1$ est un radical ; ou $R^2$ et $R^3$ sont -$COOR^9$, -$CONR^9R^{10}$, ou ensemble -CO-O-CO- ou -CO-$NR^9$-CO-, lorsque $R^1$ a les significations en tant que radical données auparavant;
ou $R^2$ et $R^3$ indépendamment l'un de l'autre sont le méthoxy ou l'éthoxy ;
$R^9$ et $R^{10}$ correspondent chacun à H, à un alkyle en $C_1$-$C_6$ ou $R^9$ et $R^{10}$ correspondent ensemble correspondent au tétraméthylène, au pentaméthylène, au 3-oxapentylène ou -$CH_2CH_2N$(alkyle en $C_1$-$C_6$)$CH_2CH_2$-, $R^{11}$ est H ou un alkyle en $C_1$-$C_4$ et n vaut 2 à 12.

**11.** Composés selon la revendication 10, où $R^4$ est H et $R^1$ signifie -$CF_3$ ou -COO(alkyle en $C_1$-$C_4$).

**12.** Composés selon la revendication 10, où $R^1$ et $R^4$ signifient H.

**13.** Composés selon la revendication 1, où $R^6$ et $R^7$ sont H et $R^1$ représente un (alkyle en $C_1$-$C_6$)-phénylthio, un (alkyle en $C_1$-$C_6$)phényloxy, un phényloxy, un alcoxy en $C_1$-$C_{12}$ ou un alkylthio en $C_1$-$C_{12}$ non substitué ou un (alkyle en $C_1$-$C_6$)phénylthio ou un phénylthio substitué par -F, -Cl, -OH, un alcoxy en $C_1$-$C_4$ ou un alkylthio en $C_1$-$C_4$.

**14.** Composés selon la revendication 1, où $R^2$ et $R^3$ en tant qu'alkyle substitué sont un alkyle en $C_1$-$C_6$ substitué par -CN ou -COO(alkyle en $C_1$-$C_{12}$).

**15.** Composés selon la revendication 1, où $R^1$ est H, $-NO_2$, $-CF_3$ ou -COO(alkyle en $C_1$-$C_4$) ; $R^4$ signifie H ; $R^3$, $R^6$ et $R^7$ sont H et $R^2$ correspond à un alcoxy avec un nombre d'atomes de carbone de 3 à 18, à un hydroxyalkyle en $C_3$-$C_{12}$, à un dihydroxyalkyle en $C_3$-$C_6$ ; un alcénoxy en $C_3$-$C_{12}$ ou un alcynoxy en $C_3$-$C_6$ où l'atome d'oxygène n'est pas lié au groupe alcène ou alcyne, $-O\{CH_2CH_2-O\}_nR^{11}$ avec n = 2 à 12, et $R^{11}$ représentant H ou un alkyle en $C_1$-$C_6$ ; un phényloxy non substitué ou substitué par -F, -Cl, un alkyle en $C_1$-$C_4$, un alcoxy en $C_1$-$C_4$ ou un alkylthio en $C_1$-$C_4$ ; $-NR^9R^{10}$ avec $R^9$ et $R^{10}$ représentant un alkyle en $C_1$-$C_6$ ou ensemble le pentaméthylène ou le 3-oxapentylène ; un (alkyle en $C_1$-$C_6$)-SO-, un (alkyle en $C_1$-$C_6$)-SO-$_2$, un phényl-SO- ou un phényl-$SO_2$- ; un alkyle en $C_1$-$C_4$ substitué par -CN ou par -COO(alkyle en $C_1$-$C_6$) ; ou -CN ou $-CF_3$ ;
$R^2$ et $R^3$ indépendamment l'un de l'autre sont un alcoxy en $C_1$-$C_2$, $-O\{CH_2CH_2\}_nOR^{11}$ avec $R^{11}$ représente H ou un alkyle en $C_1$-$C_6$ et n vaut de 2 à 12 ; ou représentent -F, -Cl, -Br ou ont les significations données auparavant pour $R^2$ ; ainsi que $R^6$ et $R^7$ indépendamment l'un de l'autre signifient H, -F, -Cl, -Br, -CN, $-CF_3$, $-Si(CH_3)_3$, $-NO_2$, un alcoxy en $C_1$-$C_{12}$ ou un -COO(alkyle en $C_1$-$C_6$), à l'exception de $R^2$ et $R^6$ ou $R^7$ étant -F et $R^1$ et $R^4$ étant H ; ou $R^1$ à $R^4$ sont H, $R^6$ ou $R^7$ représentent H et $R^7$ ou $R^6$, ou $R^6$ et $R^7$ indépendamment l'un de l'autre représentent -F, -Cl, -Br, -CN, $-NO_2$, $-CF_3$, $-Si(CH_3)_3$, $-NO_2$, -COO(alkyle en $C_1$-$C_4$) ou un alcoxy en $C_1$-$C_{12}$.

**16.** Procédé pour la préparation des composés de formule I, selon lequel on fait réagir un anhydride de l'acide naphtalène-dicarboxylique répondant à la formule II :

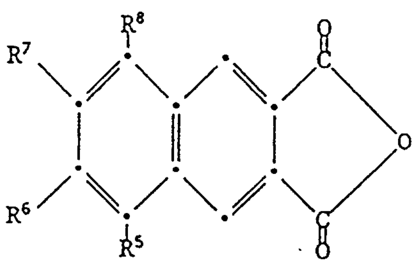

(II)

en présence d'un acide de Lewis avec un benzène de formule III :

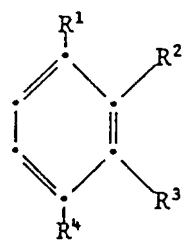

(III)

où $R^1$ à $R^8$ ont les significations données dans la revendication 1, et les composés de formule I, où au moins un de $R^1$ à $R^4$, $R^6$ et $R^7$ signifient $-NO_2$ ou un halogène, éventuellement substitué par un composé nucléophile.

**17.** Composition polymérisable sous l'action du rayonnement, contenant (a) au moins un composé polymère, oligomère ou monomère non volatil avec au moins une double liaison à insaturation éthylénique photopolymérisable ou photodimérisable et (b) au moins un composé de formule Ia en tant que photo-amorceur ou sensibilisateur,

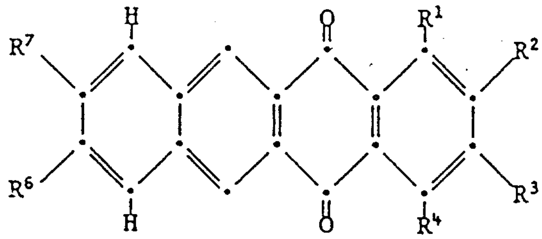

(Ia)

où au moins un de $R^1$ à $R^4$, $R^6$ et $R^7$ représente un substituant, $R^1$ et $R^4$ indépendamment l'un de l'autre représentent H, un alcoxy en $C_1$-$C_{18}$, $-O(C_mH_{2m}-O)_nR^{11}$ avec m qui vaut de 2 à 4, n qui vaut de 1 à 20 et $R^{11}$ représente H ou un alkyle en $C_1$-$C_{12}$, $R^2$, $R^3$, $R^6$ et $R^7$ représentent indépendamment l'un de l'autre H, un alcoxy en $C_1$-$C_{18}$, un (alkyle en $C_1$-$C_{18}$)-SO-, $-O(C_mH_{2m}-O)_nR^{11}$, -COO(alkyle en $C_1$-$C_{18}$),
$-COO(-C_mH_{2m}-O)_nR^{11}$ ou un alcoxy en $C_1$-$C_6$ ou un alkyle en $C_1$-$C_6$ substitué par 1 ou 2 -COO(alkyle en $C_1$-$C_{18}$) ou $-COO(C_mH_{2m}-O)_nR^{11}$, où $R^{11}$, m et n ont les significations données dans la revendication 1.

**18.** Composés selon la revendication 17, où on incorpore le composé de formule Ia en une quantité allant de 0,01 à 20 % en poids par rapport au composé photopolymérisable ou photodimérisable.

**19.** Substrat revêtu, qui est enduit sur au moins une surface d'une composition selon la revendication 17.